(19) Europäisches Patentamt
European Patent Office
Office européen des brevets

(11) Veröffentlichungsnummer: **0 332 043 B1**

# (12) EUROPÄISCHE PATENTSCHRIFT

(45) Veröffentlichungstag der Patentschrift: **18.05.94**

(51) Int. Cl.[5]: **C07D 413/10**, C07D 413/14, G03F 7/027, G03C 1/72, G03F 7/004

(21) Anmeldenummer: **89103608.9**

(22) Anmeldetag: **02.03.89**

Die Akte enthält technische Angaben, die nach dem Eingang der Anmeldung eingereicht wurden und die nicht in dieser Patentschrift enthalten sind.

## (54) 4,6-Bis-trichlormethyl-s-triazin-2-ylgruppen enthaltende Oxadiazolverbindungen, Verfahren zu ihrer Herstellung und lichtempfindliches Gemisch, das diese Verbindungen enthält.

(30) Priorität: **07.03.88 DE 3807380**

(43) Veröffentlichungstag der Anmeldung:
**13.09.89 Patentblatt 89/37**

(45) Bekanntmachung des Hinweises auf die Patenterteilung:
**18.05.94 Patentblatt 94/20**

(84) Benannte Vertragsstaaten:
**DE GB**

(56) Entgegenhaltungen:
**EP-A- 0 137 452**
**FR-A- 2 409 992**
**US-A- 4 040 922**
**US-A- 4 189 323**

(73) Patentinhaber: **HOECHST AKTIENGESELLSCHAFT**

**D-65926 Frankfurt(DE)**

(72) Erfinder: **Pawlowski, Georg, Dr.**
**Fritz-Kalle-Strasse 34**
**D-6200 Wiesbaden(DE)**
Erfinder: **Erdmann, Fritz, Dr.**
**Am Steinberg 3**
**D-6228 Eltville 4(DE)**
Erfinder: **Lutz, Heidrun**
**Martinstrasse 28-30**
**D-6500 Mainz(DE)**

**EP 0 332 043 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft 4,6-Bis-trichlormethyl-s-triazin-2-ylgruppen enthaltende Oxadiazolverbindungen, ein Verfahren zu ihrer Herstellung sowie ein lichtempfindliches Gemisch, das diese Verbindungen enthält.

Es ist bekannt, Trichlormethylgruppen enthaltende aromatische oder heterocyclische Verbindungen als Initiatoren für verschiedene photochemische Reaktionen einzusetzen.

Aus der DE-A 22 43 621 sind s-Triazine bekannt, die durch eine oder zwei Trichlormethylgruppen und eine chromophore Gruppe substituiert sind und als Photoinitiatoren in photopolymerisierbaren Gemischen sowie als Säurespender im Gemisch mit durch Säure spaltbaren Acetalen geeignet sind. Unter diesen Verbindungen sind auch solche, die im sichtbaren Bereich des elektromagnetischen Spektrums absorbieren und als Photoinitiatoren wirksam sind.

Ähnliche Verbindungen, in denen als chromophore Gruppe ein mindestens zweikerniger aromatischer Rest unmittelbar an den Triazinring gebunden ist, sind aus der DE-A 27 18 259 (= US-A 4 189 323) bekannt.

In der EP-A 137 452 sind ähnliche 4,6-Bis-trichlormethyl-s-triazine beschrieben, die in der 2-Stellung eine ggf. substituierte Styrylgruppe tragen. Die Absorptionsmaxima dieser Verbindungen liegen meistens im nahen UV-Bereich.

In der DE-A 28 51 472 sind lichtempfindliche Gemische beschrieben, die als Photoinitiatoren 2-Halogenmethyl-5-vinyl-1,3,4-oxadiazolderivate enthalten.

Ähnliche Verbindungen, die als Photoinitiatoren wirksam sind, sind aus der DE-A 35 06 274 bekannt. Diese Verbindungen weisen Absorptionsmaxima bei längeren Wellenlängen auf.

Aus den DE-A 30 21 590 und 30 21 599 sind mit Trichlormethylphenylgruppen substituierte Halogenoxazole bekannt, die ebenso wie alle zuvor genannten Verbindungen als Photoinitiatoren geeignet sind.

In der US-A 4 040 922 werden flüssige photopolymerisierbare Gemische beschrieben, die durch UV-Strahlung gehärtet werden und damit als lösemittelfreie Lacke geeignet sind. Sie enthalten als Photoinitiatoren u.a. 2,5-Bis-(4-halogenmethyl-phenyl)-oxadiazole.

Schließlich sind aus den EP-A 135 348 und 135 863 1-Alkyl-2-carbonylmethylenbenzthiazole und ähnliche Heterocyclen bekannt, die an der Carbonylgruppe eine Trichlormethylphenylgruppe tragen. Auch diese Verbindungen haben ihre höchste Empfindlichkeit im nahen UV-Bereich.

Die Reaktionsbedingungen zur Herstellung vieler dieser Verbindungen sind verhältnismäßig drastisch, so daß die Ausbeute relativ gering ist und die Bildung von unerwünschten, schwer abtrennbaren Nebenprodukten begünstigt wird (z. B. DE-A 22 43 621, 27 18 259 oder 28 51 472). Bei vielen bekannten Initiatoren erfordert die unzureichende Empfindlichkeit, daß verschiedene Initiatorsysteme miteinander kombiniert werden. Darüber hinaus hat es sich erwiesen, daß gerade die empfindlichsten der bekannten Initiatoren keine den Anforderungen der Praxis entsprechende Lagerfähigkeit in lichtempfindlichen Gemischen, insbesondere im Kontakt mit Kupferoberflächen aufweisen. Schließlich weisen auch die meisten der bekannten Photoinitiatoren, die auch als photolytisch aktivierbare Säurespender eingesetzt werden können, Empfindlichkeitsmaxima zwischen 380 und 420 nm auf.

In der modernen Belichtungstechnologie zeichnen sich jedoch zwei neue, gegenläufige Trends ab. Zum einen läßt sich beobachten, daß zur Erzeugung feinerer Strukturen und deren verbesserter Wiedergabe insbesondere in der Mikroelektronik in der Zukunft vermehrt Lichtquellen genutzt werden, deren Emissionsmaxima bei kürzeren Wellenlängen, z. B. bei 365 nm, liegen. Auf der anderen Seite läßt sich in der graphischen Industrie beobachten, daß im Zuge der Digitalisierung der Informationen Lichtquellen, z. B. Laser, Verwendung finden, die im sichtbaren Bereich des elektromagnetischen Spektrums, i.a. oberhalb 450 nm emittieren.

Aufgabe der vorliegenden Erfindung ist es, neue lichtempfindliche Verbindungen bereitzustellen, die sich in verschiedenartigen lichtempfindlichen Materialien einsetzen lassen, die relativ einfach zugänglich sind und bei ihrem Einsatz eine große Breite von Variationsmöglichkeiten bieten, um den Bedürfnissen auf den verschiedenen Anwendungsgebieten jeweils optimal angepaßt werden zu können. Insbesondere ist es die Aufgabe der vorliegenden Erfindung, lichtempfindliche Verbindungen bereitzustellen, die im UV-Spektralbereich um 365 nm hohe Empfindlichkeiten aufweisen, so daß ihre Aktivität bereits durch schwache Lichtquellen angeregt wird oder eine Verkürzung der Belichtungszeit möglich ist. Weiterhin sollen die lichtempfindlichen Gemische für den Spektralbereich zwischen 400 und 700, insbesondere von 450 bis 650 nm durch den Zusatz geeigneter Farbstoffe oder Sensibilisatoren zu hohen Empfindlichkeiten sensibilisiert werden können. Insbesondere bei lichtempfindlichen Gemischen hoher Schichtdicke ist es notwendig, daß der Photoinitiator keine hohe Eigenfärbung aufweist, um eine durchgehende Photoreaktion zu ermöglichen. Ferner sollen die lichtempfindlichen Gemische, die die Initiatoren enthalten, unabhängig vom Material des Schichtträgers eine hohe Lagerstabiltät aufweisen und nach der Bestrahlung einen deutlich sichtbaren

Bildkontrast in der lichtempfindlichen Schicht ergeben.

Die Aufgabe wird gelöst durch Verbindungen der allgemeinen Formel I

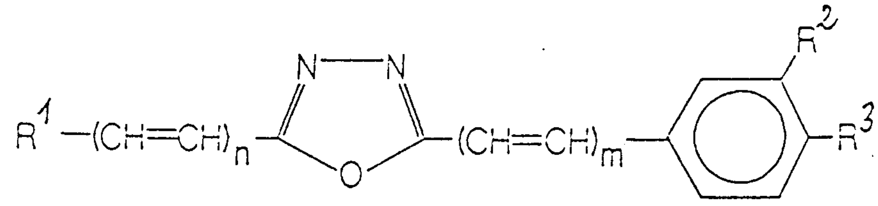

(I)

worin

| | |
|---|---|
| $R^1$ | einen Phenyl-, Naphthyl- oder einen 1- bis 3-kernigen aromatischen N-Heteroylrest, der unsubsituiert oder durch 1 bis 3 Alkyl- oder Alkoxyreste mit 1 bis 10 Kohlenstoffatomen, Perhalogenalkylreste mit 1 bis 6 Kohlenstoffatomen, Nitro-, Cyano, Carboxyl-, Carbonyl-, Diphenylamino- oder Phenylreste substituiert ist, und |
| $R^2$ und $R^3$ | voneinander verschieden sind und entweder ein Wasserstoffatom oder eine 4,6-Bis-trichlormethyl-s-triazin-2-yl-gruppe und |
| n und m | voneinander unabhängig die Zahlen 0 oder 1 |

bedeuten.

Erfindungsgemäß wird ferner ein lichtempfindliches Gemisch vorgeschlagen, das eine lichtempfindliche organische Verbindung (a) mit mindestens einem 4,6-Bis-trichlormethyl-s-triazin-2-ylsubstituenten und eine Verbindung (b) enthält, die mit dem Lichtreaktionsprodukt der Verbindung (a) unter Ausbildung eines Produktes zu reagieren vermag, das eine von (b) unterschiedliche Lichtabsorption, Klebrigkeit oder Löslichkeit in einem Entwickler aufweist.

Das erfindungsgemäße Gemisch ist dadurch gekennzeichnet, daß die Verbindung (a) eine Verbindung gemäß der oben angegebenen Formel I ist.

Die erfindungsgemäßen Verbindungen bilden unter Einwirkung von aktinischer Strahlung freie Radikale, die zur Einleitung chemischer Reaktionen, insbesondere von radikalisch initiierten Polymerisationen, befähigt sind. Sie bilden ferner bei Bestrahlung Halogenwasserstoff, durch den säurekatalysierte Reaktionen, z. B. die Spaltung von Acetalbindungen, oder Salzbildungen, z. B. Farbumschläge von Indikatorfarbstoffen, in Gang gesetzt werden können.

Wenn $R^1$ durch Alkyl- oder Alkoxyreste substituiert ist, so können diese bevorzugt 1 bis 6 Kohlenstoffatome enthalten. Sie können geradkettig oder verzweigt oder ggf. zu einem cycloaliphatischen Rest, z. B. einem Cyclohexylrest, ringgeschlossen sein.

Die erfindungsgemäßen Verbindungen lassen sich auf zwei Wegen in vorteilhafter Weise darstellen.

Nach einem ersten, nicht bevorzugten Verfahren, welches durch nachstehendes Reaktionsschema erläutert wird und in dem $R^1$, $R^2$, $R^3$, m und n die vorstehend beschriebene Bedeutung aufweisen

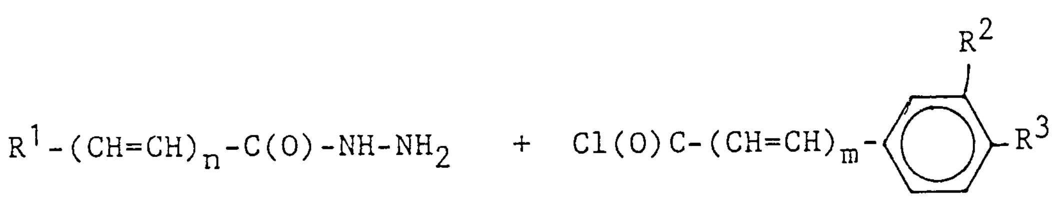

(IV) (III)

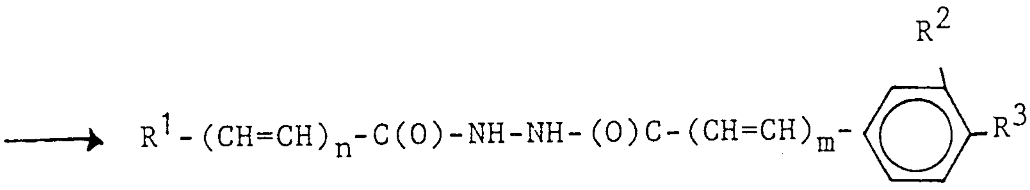

(V)

I

lassen sich Säurehydrazide der Formel IV mit Säurechloriden der Formel III zu den Bisacylhydraziden der Formel V umsetzen, die dann durch ein Entwässerungsmittel in die erfindungsgemäßen Verbindungen der Formel I umgewandelt werden. Die Hydrazide lassen sich nach bekannten Verfahren, beispielsweise nach W.O. Godtfredsen und S. Vangedal, Acta Chem. Scand., 9, 1498 (1955) oder R. Harada und H. Kondo, Bull. Chem. Soc. Jpn., 41, 2521 (1968), herstellen. Weitere Herstellungsmethoden sind in Houben-Weyl "Methoden der Organischen Chemie" Bd. VIII, S. 676 ff beschrieben. Die Hydrazide der Formel IV werden nach bekannten Verfahren, die z. B. in Houben-Weyl Bd. 10/2, S. 127 ff referiert werden, durch Umsetzung mit den Säurechloriden der Formel III in die Bisacylhydrazide der Formel V umgewandelt. Die Herstellung der Säurechloride der Formel III ist in der gleichzeitig eingereichten EP-A 03 32 042 beschrieben. Die Umwandlung der Bisacylhydrazide der Formel V in die erfindungsgemäßen Oxadiazolderivate der Formel I kann nach dem Verfahren von M.P. Hutt, E.F. Elslager und L.M. Werbel, J. Heterocycl. Chem., 7, 511 (1970) erfolgen.

In einem zweiten, bevorzugten Verfahren werden aromatische Nitrile der Formel VI durch Natrium-, bevorzugt Lithium- oder Ammoniumazid in die entsprechenden Tetrazole der Formel II übergeführt. Dies kann beispielsweise analog zu der Vorschrift von W.G. Finnegan, R.A. Henry und R. Lofquist, J. Amer. Chem. Soc., 80, 3908 (1958) erfolgen. Die Tetrazole der Formel II lassen sich dann mit den Säurechloriden der Formel III in einer Einstufenreaktion in hohen Ausbeuten zu den erfindungsgemäßen Verbindungen der Formel I umwandeln, was beispielsweise analog zu den Vorschriften von R. Huisgen, J. Sauer und H.J. Sturn, Angew. Chem., 70, 272 (1958) geschehen kann:

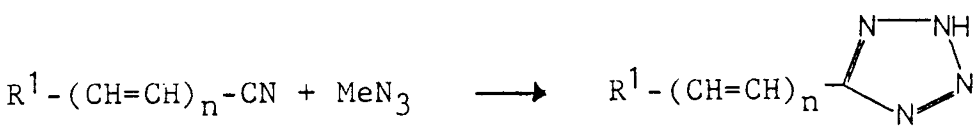

(VI) (II)

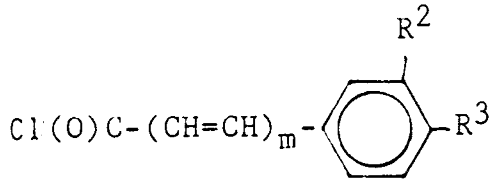

II + (III)

I

Der Ablauf der Reaktion nach diesem Schema ist an sich überraschend, da bekannt ist, daß Trichlormethylgruppen, insbesondere wenn sie wie im vorliegenden Fall mit einem Triazinring verknüpft sind, überaus leicht nukleophile Substitutionen, insbesondere mit Stickstoff enthaltenden Nukleophilen, eingehen. Aus den nachfolgend beschriebenen Beispielen ist jedoch ersichtlich, daß diese mögliche Nebenreaktion praktisch keine Rolle spielt und die erfindungsgemäßen Verbindungen der Formel I in hohen Ausbeuten anfallen.

Als Nitrile der allgemeinen Formel VI lassen sich Alkylnitrile und insbesondere Aryl- oder Arylethenylnitrile verwenden, die gegebenenfalls durch inerte Reste substituiert sind, die keine oder nur unter drastischen Bedingungen 1,3-dipolare Additionen eingehen. Derartige Nitrile lassen sich auf mannigfaltige bekannte Weise herstellen.

Die Überführung in die Tetrazole der Formel II erfolgt zweckmäßig derart, daß das Nitril mit der 1- bis 5-fachen, bevorzugt etwa 2-fachen stöchiometrischen Menge eines Ammonium-, Lithium- oder Natriumazids in einem polaren Lösungsmittel, bevorzugt Dimethylformamid oder 2-Methoxy-ethanol, in einem Temperaturbereich zwischen 50 und 200 °C, bevorzugt von 100 bis 120 °C reagieren gelassen wird. Wenn als Reaktionsteilnehmer Natriumazid verwendet wird, so ist es sinnvoll, der Mischung ein Lithium- oder Ammoniumsalz, z. B. Lithium- oder Ammoniumchlorid, zuzufügen, da das intermediär gebildete Lithiumazid eine bessere Löslichkeit in den bei dem Verfahren verwendbaren Lösungsmitteln aufweist. Die Reaktionsmischung wird 5 bis 100, bevorzugt 20 bis 30 Stunden bei der vorgegebenen Temperatur gehalten und das Gemisch nach Abkühlung auf Wasser gegossen. Nach vorsichtigem Ansäuern mit wäßriger Salzsäure fällt das Tetrazol der Formel II, gegebenenfalls nach ausreichender Kühlung, in hoher Reinheit und Ausbeute aus und kann durch Filtration isoliert werden. War die Umsetzung nicht quantitativ, so läßt sich unumgesetztes Nitril durch Filtrieren oder Extrahieren der wäßrigen, nicht angesäuerten Lösung mit einem organischen Lösungsmittel, z. B. Ether oder Dichlormethan, entfernen.

Zur Umwandlung in die erfindungsgemäßen Oxadiazolderivate der Formel I werden die Tetrazole in der 5- bis 50-fachen Menge einer Base, bevorzugt Pyridin, gelöst oder suspendiert. Dazu wird das Säurechlorid der allgemeinen Formel III gegeben, wobei die Mischung bei Bedarf gekühlt werden sollte. Das Gemisch wird dann langsam auf 50 bis 150 °C, bevorzugt 80 bis 120 °C erhitzt, wobei eine stark einsetzende Stickstoffentwicklung beobachtet wird. Nach 1 bis 5 Stunden, bevorzugt 2 bis 3 Stunden wird die Mischung, sofern die Stickstoffentwicklung beendet ist, abkühlen gelassen und auf Wasser gegossen, wobei gewöhnlich das erfindungsgemäße Oxadiazolderivat der Formel I in hoher Reinheit anfällt. Bei Bedarf kann die wäßrige Lösung angesäuert und/oder mit einem organischen Lösungsmittel extrahiert werden. In diesem Fall erfolgt die Isolierung durch Einengen des Lösungsmittels. Die erfindungsgemäßen Verbindungen können gegebenenfalls durch Umkristallisation weiter gereinigt werden.

Die erfindungsgemäßen Verbindungen sind als Photoinitiatoren für photopolymerisierbare Gemische geeignet, die als wesentliche Bestandteile polymerisierbare Verbindungen, Initiatoren und ggf. Bindemittel enthalten.

Die in den erfindungsgemäßen lichtempfindlichen Gemischen verwendeten polymerisierbaren Verbindungen enthalten mindestens eine ethylenische Doppelbindung und können als Monomere, Oligomere, Polymere oder Gemische aus diesen Komponenten vorliegen. Beispiele für geeignete Verbindungen sind ggf. mehrfach ungesättigte Carbonsäuren, deren Salze, Säurederivate, wie Ester oder Amide, Derivate, die sich von der Kohlensäure ableiten, z. B. Urethane, Sulfonyl- oder Phosphinylurethane oder die entsprechenden Harnstoffverbindungen, ungesättigte Ether sowie ungesättigte Derivate, die sich von Epoxiden ableiten lassen.

Beispiele für Carbonsäuren sind Acrylsäure, Methacrylsäure, Itaconsäure, Crotonsäure, Isocrotonsäure und Maleinsäure. Beispiele für Carbonsäuresalze sind die Natrium- und Kaliumsalze der vorstehend genannten Carbonsäuren.

Als Ester von ungesättigten Carbonsäuren mit ggf. mehrwertigen Alkoholen sind Acryl- und Methacrylsäureester, wie Ethylenglykoldi(meth)acrylat, Triethylenglykoldi(meth)acrylat, 1,3-Butandioldi(meth)acrylat, Tetramethylendioldi(meth)acrylat, Propylenglykoldi(meth)acrylat, Trimethylolpropantri(meth)acrylat, Trimethylolethantri(meth)acrylat, 1,4-Cyclohexandioldi(meth)acrylat, Tetraethylenglykoldi(meth)acrylat, Pentaerythritdi(meth)acrylat, Pentaerythrittri(meth)acrylat, Pentaerythrittetra(meth)acrylat, Dipentaerythritdi-(meth)acrylat, Dipentaerythrittetra(meth)acrylat, Sorbittri(meth)acrylat, Sorbittetra(meth)acrylat, Sorbitpenta-(meth)acrylat, Sorbithexa(meth)acrylat und Polyester(meth)acrylat-Oligomere, 2,2-Bis-[p-(3-(meth)-acryloyloxy-2-hydroxy-propoxy)phenyl]-propan und 2,2-Bis-[4-(meth)acryloyloxyethoxy-phenyl]-propan; Itaconsäureester, wie Ethylenglykoldiitaconat, Propylenglykoldiitaconat, 1,3-Butandioldiitaconat, 1,4-Butandioldiitaconat, Tetramethylendioldiitaconat, Pentaerythritdiitaconat und Sorbittetraitaconat; Crotonsäureester, wie Ethylenglykoldicrotonat, Tetramethylendioldicrotonat, Pentaerythritdicrotonat und Sorbittetracrotonat; Isocrotonsäureester, wie Ethylenglykoldiisocrotonat, Pentaerythritdiisocrotonat und Sorbittetraisocrotonat; sowie Maleinsäureester, wie Ethylenglykoldimaleinat, Triethylenglykoldimaleinat, Pentaerythritdimaleinat und Sorbittetramaleinat geeignet. Diese Ester können einzeln oder als Gemische eingesetzt werden.

Als Amide von ungesättigten Carbonsäuren mit ggf. mehrwertigen Aminen sind Acryl- und Methacrylamide, z. B. Methylen-bis-(meth)acrylamid, 1,6-Hexamethylen-bis-(meth)acrylamid, Diethylentriamin-tris-(meth)acrylamid und Xylylen-bis-(meth)acrylamid verwendbar.

Weitere mit Vorteil verwendbare polymerisierbare Verbindungen mit einer ethylenisch ungesättigten Bindung sind Vinylurethanverbindungen mit zwei oder mehreren polymerisierbaren Vinylgruppen im Molekül, die durch Additionsreaktion eines Hydroxyalkyl(meth)acrylats, z. B. Hydroxyethylmethacrylat oder 2-Hydroxy-propylacrylat, mit einem Isocyanat mit mindestens zwei Isocyanatgruppen im Molekül erhalten werden, oder solche, die durch Additionsreaktion aus einem Isocyanatoalkyl(meth)acrylat, z. B. 2-Isocyanatoethylmethacrylat, und einem mehrwertigen, gegebenenfalls Stickstoffatome enthaltenden Alkohol erhalten werden.

Unter den aufgeführten Verbindungen stellen die Acryl-und Methacrylsäureester mehrwertiger Alkohole sowie die Umsetzungsprodukte von Diisocyanaten mit Partialestern mehrwertiger ungesättigter Alkohole und die Umsetzungsprodukte von Hydroxyalkyl(meth)acrylaten mit Polyisocyanaten und Isocyanatoalkyl-(meth)acrylaten mit Polyalkoholen besonders bevorzugte polymerisierbare Komponenten dar. Beispiele für die zuletzt genannten Monomeren sind in den DE-A 20 64 079, 23 61 041 und 28 22 190 beschrieben. Der Mengenanteil der Schicht an Monomeren beträgt im allgemeinen etwa 10 bis 80, vorzugsweise 20 bis 60 Gew.-%, bezogen auf die Menge der nichtflüchtigen Bestandteile.

Beim Einsatz der erfindungsgemäßen Photoinitiatoren in photopolymerisierbaren Gemischen können diese ferner ein Bindemittel enthalten. Das Bindemittel muß mit der ethylenisch ungesättigten polymerisierbaren Verbindung und dem erfindungsgemäßen Photoinitiator verträglich sein. Die lichtempfindliche Schicht muß nach der bildmäßigen Belichtung durch Auswaschen oder Auseinanderziehen (peel apart) verarbeitbar sein. Außerdem sollte das Bindemittel der lichtempfindlichen Schicht ausreichende Zähigkeit, Festigkeit, Abriebbeständigkeit und Flexibilität verleihen. Gewöhnlich ist das Bindemittel ein lineares organisches Polymeres.

Beispiele für verwendbare Bindemittel sind chloriertes Polyethylen, chloriertes Polypropylen, Poly-(meth)acrylsäurealkylester, bei denen die Alkylgruppe z. B. Methyl, Ethyl, n-Butyl, i-Butyl, n-Hexyl oder 2-Ethylhexyl ist, Copolymere der genannten (Meth)acrylsäurealkylester mit mindestens einem Monomeren, wie Acrylnitril, Vinylchlorid, Vinylidenchlorid, Styrol oder Butadien; Polyvinylchlorid, Vinylchlorid/ Acrylnitril-Copolymere, Polyvinylidenchlorid, Vinylidenchlorid/Acrylnitril-Copolymere, Polyvinylacetat, Polyvinylalkohol, Polyacrylnitril, Acrylnitril/Styrol-Copolymere, Acrylnitril/Butadien/Styrol-Copolymere, Polystyrol, Polymethylstyrol, Polyamide (z. B. Nylon-6), Polyurethane, Methylcellulose, Ethylcellulose, Acetylcellulose, Polyvinylformal und Polyvinylbutyral.

Besonders geeignet sind Bindemittel, die in Wasser unlöslich, in organischen Lösungsmitteln löslich und in wäßrig-alkalischen Lösungen löslich oder zumindest quellbar sind.

Besonders erwähnt werden sollen Carboxylgruppen enthaltende Bindemittel, z. B. Copolymerisate aus (Meth)acrylsäure und/oder deren ungesättigten Homologen, wie Crotonsäure, Copolymerisate des Maleinsäureanhydrids oder seiner Halbester, Umsetzungsprodukte hydroxylgruppenhaltiger Polymerer mit Dicarbonsäureanhydriden sowie deren Mischungen.

Weiterhin sind geeignet Umsetzungsprodukte aus Polymeren, die H-acide Gruppen tragen, welche ganz oder teilweise mit aktivierten Isocyanaten umgesetzt wurden, z. B. Umsetzungsprodukte aus hydroxylgruppenhaltigen Polymeren mit aliphatischen oder aromatischen Sulfonylisocyanaten oder Phosphinsäureisocyanaten.

Darüber hinaus sind geeignet: Hydroxylgruppen enthaltende Polymere, z. B. Copolymere von Hydroxyalkyl(meth)acrylaten, Copolymere des Allylalkohols, Copolymere des Vinylalkohols, Polyurethane oder Polyester, sowie Epoxyharze, sofern sie eine ausreichende Anzahl von freien OH-Gruppen tragen, oder derart modifiziert sind, daß sie in wäßrig-alkalischen Lösungen löslich sind, oder solche Polymere, die aromatisch gebundene Hydroxylgruppen tragen, wie Kondensationsprodukte von kondensationsfähigen Carbonylverbindungen, insbesondere Formaldehyd, Acetaldehyd oder Aceton, mit Phenolen oder Copolymerisate der Hydroxystyrole. Schließlich lassen sich auch Copolymerisate des (Meth)acrylsäureamids mit Alkyl(meth)acrylaten verwenden.

Die vorstehend beschriebenen Polymeren sind insbesondere dann geeignet, wenn sie ein Molekulargewicht zwischen 500 und 200.000 oder darüber, bevorzugt 1.000 bis 100.000 aufweisen und entweder Säurezahlen zwischen 10 und 250, bevorzugt von 20 bis 200, oder Hydroxylzahlen zwischen 50 und 750, bevorzugt von 100 bis 500, aufweisen.

Als bevorzugte alkalilösliche Bindemittel seien nachstehend erwähnt:

Copolymerisate der (Meth)acrylsäure mit Alkyl(meth)acrylaten, (Meth)acrylsäurenitril oder dergleichen, Copolymerisate der Crotonsäure mit Alkyl(meth)acrylaten, (Meth)acrylsäurenitril oder dergleichen, Copolymerisate der Vinylessigsäure mit Alkyl(meth)acrylaten, Copolymerisate des Maleinsäureanhydrids mit ggf. substituierten Styrolen, ungesättigten Kohlenwasserstoffen, ungesättigten Ethern oder Estern, Veresterungsprodukte der Copolymerisate des Maleinsäureanhydrids, Veresterungsprodukte von Hydroxylgruppen enthaltenden Polymeren mit Anhydriden von Di- oder Polycarbonsäuren, Copolymerisate der Hydroxyalkyl-

(meth)acrylate mit Alkyl(meth)acrylaten, (Meth)acrylsäurenitril oder dergleichen, Copolymerisate des Allylalkohols mit ggf. substituierten Styrolen, Copolymerisate des Vinylalkohols mit Alkyl(meth)acrylaten oder anderen polymerisationsfähigen ungesättigten Verbindungen, Polyurethane, sofern sie eine ausreichende Anzahl freier OH-Gruppen aufweisen, Epoxyharze, Polyester, teilverseifte Vinylacetat-Copolymere, Polyvinylacetale mit freien OH-Gruppen, Copolymerisate der Hydroxystyrole mit Alkyl(meth)acrylaten oder dergleichen, Phenol-Formaldehyd-Harze, z. B. Novolake.

Die Menge des Bindemittels in der lichtempfindlichen Schicht beträgt im allgemeinen 20 bis 90, vorzugsweise 40 bis 80 Gew.-%.

Die erfindungsgemäßen Photoinitiatoren werden derartigen Gemischen in Mengen zwischen 0,1 und 15,0, bevorzugt von 0,2 bis 5 Gew.-% zugemischt.

Die photopolymerisierbaren Gemische können je nach geplanter Anwendung und je nach den gewünschten Eigenschaften verschiedenartige Stoffe als Zusätze enthalten. Beispiele sind:
Inhibitoren zur Verhinderung der thermischen Polymerisation, Wasserstoffdonatoren, Regler für die spektrale Empfindlichkeit, Farbstoffe, gefärbte und ungefärbte Pigmente, Farbbildner, Indikatoren und Weichmacher.

Geeignete Inhibitoren sind z. B. Hydrochinon, p-Methoxyphenol, Di-t-Butyl-p-kresol, Pyrogallol, t-Butylbrenzkatechin, Benzochinon, Kupfer-I-chlorid, Phenothiazin, Chloranil, Naphthylamin, Naphthol, Nitrobenzol und Dinitrobenzol.

Geeignete Farbstoffe oder Pigmente sind z. B. Methylenblau, Kristallviolett, Rhodamin B, Fuchsin, Aurin, Azofarbstoffe, Anthrachinonfarbstoffe, Titandioxid, Ruß, Eisenoxid, Phthalocyaninpigmente oder Azopigmente, wobei darauf zu achten ist, daß die Absorption des eingesetzten Farbstoffs im Anregungsbereich des Photoinitiators nicht zu stark ist.

Beispiele für Weichmacher sind Phthalsäureester, Glykolester, Phosphorsäureester oder aliphatische Dicarbonsäureester.

Das Gemisch kann außerdem einen Sensibilisator und/oder einen weiteren Photoinitiator enthalten, die so ausgewählt werden, daß die Photopolymerisationsgeschwindigkeit erhöht wird, wenn sie zusammen mit dem Photoinitiator der allgemeinen Formel I verwendet werden. Verwendbare Sensibilisatoren sind Benzoin, Benzoinalkylether, 9-Fluorenon, 2-Brom-9-anthron, 2-Ethyl-9-anthron, 9,10-Anthrachinon, substituierte Anthrachinone, Xanthon, substituierte Xanthone, Thioxanthon, Benzil, Dibenzalaceton, substituierte Chalkone, Benzophenon oder Benzanthron, Eosin- oder Fluoresceinderivate, Acridine, Pyronine oder ähnliche.

Verwendbare Coinitiatoren sind insbesondere Trichlormethylgruppen enthaltende Photoinitiatoren, deren Absorptionsmaximum deutlich oberhalb der Absorptionsmaxima der erfindungsgemäßen Photoinitiatoren liegt. Besonders geeignet sind z. B. die in der gleichzeitig eingereichten Patentanmeldung P 38 07 381.1 beschriebenen 4,6-Bis-trichlormethyl-s-triazin-2-ylbenzoylmethylenheterocyclen. Durch diese Maßnahmen läßt sich die spektrale Empfindlichkeit des lichtempfindlichen Gemischs über einen weiten Wellenlängenbereich ausdehnen.

Das photopolymerisierbare Gemisch kann für die verschiedensten Anwendungen Einsatz finden, beispielsweise zur Herstellung von Sicherheitsglas, von Lacken, die durch Licht oder Korpuskularstrahlen, z. B. Elektronenstrahlen, gehärtet werden, auf dem Dentalgebiet und insbesondere als lichtempfindliches Kopiermaterial auf dem Reproduktionsgebiet. Als Anwendungsmöglichkeiten auf diesem Gebiet seien genannt: Kopierschichten für die photomechanische Herstellung von Druckformen für den Hochdruck, den Flexodruck, den Flachdruck, den Tiefdruck, den Siebdruck, von Reliefkopien, z. B. Herstellung von Texten in Blindenschrift, von Einzelkopien, Gerbbildern, Pigmentbildern usw.. Weiter sind die Gemische zur photomechanischen Herstellung von Ätzreservagen, z. B. für die Fertigung von Namensschildern, von gedruckten Schaltungen und für das Formteilätzen, anwendbar.

Die gewerbliche Verwertung des Gemischs für die genannten Anwendungszwecke kann in der Form einer flüssigen Lösung oder Dispersion, z. B. als Photoresistlösung, erfolgen, die vom Verbraucher selbst auf einen individuellen Träger, z. B. zum Formteilätzen, für die Herstellung gedruckter Schaltungen, von Siebdruckschablonen und dgl., aufgebracht wird. Das Gemisch kann auch als feste lichtempfindliche Schicht auf einem geeigneten Träger in Form eines lagerfähig vorbeschichteten lichtempfindlichen Kopiermaterials, z. B. für die Herstellung von Druckformen, vorliegen. Ebenso ist es für die Herstellung von Trockenresist geeignet.

Es ist im allgemeinen günstig, die Gemische während der Lichtpolymerisation dem Einfluß des Luftsauerstoffes weitgehend zu entziehen. Im Fall der Anwendung des Gemischs in Form dünner Kopierschichten ist es empfehlenswert, einen geeigneten, für Sauerstoff wenig durchlässigen Deckfilm aufzubringen. Dieser kann selbsttragend sein und vor der Entwicklung der Kopierschicht abgezogen werden. Für diesen Zweck sind z. B. Polyesterfilme geeignet. Der Deckfilm kann auch aus einem Material bestehen, das sich in der Entwicklerflüssigkeit löst oder mindestens an den nicht gehärteten Stellen bei der Entwicklung

entfernen läßt. Hierfür geeignete Materialien sind z. B. Wachse, Polyamide, Polyvinylalkohol, Polyphosphate, Zucker usw..

Als Schichtträger für mit dem erfindungsgemäßen Gemisch hergestellte Kopiermaterialien sind beispielsweise Aluminium, Stahl, Zink, Kupfer und Kunststoff-Folien, z. B. aus Polyethylenterephthalat oder Cellulosacetat, sowie Siebdruckträger, wie Perlongaze, geeignet.

Weiterhin können die erfindungsgemäßen Verbindungen auch in solchen strahlungsempfindlichen Gemischen eingesetzt werden, deren Eigenschaftsänderung durch saure Katalysatoren, die bei der Photolyse des Initiators entstehen, eingeleitet wird. Zu nennen sind etwa die kationische Polymerisation von Systemen, die Vinylether, N-Vinylverbindungen wie N-Vinylcarbazol oder spezielle säurelabile Lactone enthalten, wobei nicht ausgeschlossen wird, daß bei einigen dieser Reaktionen auch radikalische Vorgänge beteiligt sind. Als durch Säuren härtbare Massen sind weiterhin Aminoplaste wie Harnstoff/Formaldehydharze, Melamin/Formaldehydharze und andere N-Methylolverbindungen sowie Phenol/Formaldehydharze zu nennen. Wenn auch die Härtung von Epoxyharzen im allgemeinen durch Lewis-Säuren bzw. solche Säuren erfolgt, deren Anionen eine geringere Nukleophilie als Chlorid besitzen, also als das Anion der bei der Photolyse der neuen Verbindungen entstehenden Halogenwasserstoffsäure, so härten doch Schichten, die aus Epoxyharzen und Novolaken bestehen, bei Belichtung in Gegenwart der erfindungsgemäßen Verbindungen glatt aus.

Eine weitere vorteilhafte Eigenschaft der neuen Verbindungen besteht in ihrer Fähigkeit, in gefärbten Systemen bei der Photolyse Farbumschläge hervorzurufen; aus Farbvorläufern, z. B. Leukoverbindungen, Farbbildung zu induzieren oder bathochrome Farbverschiebungen und -vertiefungen in Gemischen zu bewirken, die Cyanin-, Merocyaninoder Styrylfarbbasen enthalten. Auch kann z. B. in den in der DE-A 15 72 080 beschriebenen Gemischen, die Farbbase, N-Vinylcarbazol und einen Halogenkohlenwasserstoff enthalten, die Halogenverbindung Tetrabrommethan durch einen Bruchteil ihrer Menge an erfindungsgemäßer Verbindung ersetzt werden. Farbumschläge sind in der Technik auch z. B. bei der Herstellung von Druckformen erwünscht, um nach der Belichtung bereits vor der Entwicklung das Kopierergebnis beurteilen zu können.

Statt der in den DE-A 23 31 377 und 26 41 100 genannten Säurespender sind vorteilhaft die vorliegenden Verbindungen zu benutzen.

Ein besonders bevorzugtes Anwendungsgebiet für die erfindungsgemäßen Verbindungen sind Gemische, die neben ihnen als wesentliche Komponente eine Verbindung mit mindestens einer durch Säure spaltbaren C-O-C-Gruppierung enthalten. Als durch Säure spaltbare Verbindungen sind in erster Linie zu nennen:

A) solche mit mindestens einer Orthocarbonsäureester-und bzw. oder Carbonsäureamidacetalgruppierung, wobei die Verbindungen auch polymeren Charakter haben und die genannten Gruppierungen als verknüpfende Elemente in der Hauptkette oder als seitenständige Substituenten auftreten können,

B) Polymerverbindungen mit wiederkehrenden Acetal- und/oder Ketalgruppierungen und

C) Polymerverbindungen mit wiederkehrenden Einheiten von aktivierten Estern der Kohlensäure.

Durch Säure spaltbare Verbindungen des Typs A als Komponenten strahlungsempfindlicher Gemische sind in den DE-A 26 10 842 oder 29 28 636 ausführlich beschrieben; Gemische, die Verbindungen des Typs B enthalten, sind Gegenstand der DE-C 27 18 254, und Gemische, die Verbindungen des Typs C enthalten, sind in der EP-A 102 450 beschrieben.

Als durch Säure spaltbare Verbindungen sind z. B. auch die speziellen Aryl-alkyl-acetale und -aminale der DE-C 23 06 248 zu nennen, die durch die Photolyseprodukte der erfindungsgemäßen Verbindungen ebenfalls abgebaut werden.

Solche Gemische, in denen durch Einwirkung von aktinischer Strahlung mittelbar oder unmittelbar Moleküle in kleinere umgewandelt werden, weisen an den bestrahlten Stellen im allgemeinen eine erhöhte Löslichkeit, Klebrigkeit oder Flüchtigkeit auf. Diese Partien können durch geeignete Maßnahmen, beispielsweise Herauslösen mit einer Entwicklungsflüssigkeit, entfernt werden. Bei Kopiermaterialien spricht man in diesen Fällen von positiv arbeitenden Systemen.

Die bei vielen Positiv-Kopiermaterialien bewährten Novolak-Kondensationsharze haben sich als Zusatz auch bei der Anwendung der erfindungsgemäßen Verbindungen in Gemischen mit durch Säure spaltbaren Verbindungen als besonders brauchbar und vorteilhaft erwiesen. Sie fördern die starke Differenzierung zwischen den belichteten und unbelichteten Schichtpartien beim Entwickeln, besonders die höher kondensierten Harze mit substituierten Phenolen als Formaldehyd-Kondensationspartner. Die Art und Menge der Novolak-Harze kann je nach Anwendungszweck verschieden sein; bevorzugt sind Novolak-Anteile am Gesamtfeststoff zwischen 30 und 90, besonders bevorzugt 55 - 85 Gew.-%.

Zusätzlich können noch zahlreiche andere Harze mitverwendet werden, bevorzugt Vinylpolymerisate wie Polyvinylacetate, Polyacrylate, Polyvinylether und Polyvinylpyrrolidone, die selbst durch Comonomere

modifiziert sein können. Der günstigste Anteil an diesen Harzen richtet sich nach den anwendungstechnischen Erfordernissen und dem Einfluß auf die Entwicklungsbedingungen und beträgt im allgemeinen nicht mehr als 20 % vom Novolak. In geringen Mengen kann das lichtempfindliche Gemisch für spezielle Erfordernisse wie Flexibilität, Haftung und Glanz etc. außerdem noch Substanzen wie Polyglykole, Cellulose-Derivate wie Ethylcellulose, Netzmittel, Farbstoffe und feinteilige Pigmente sowie bei Bedarf UV-Absorber enthalten. Entwickelt wird vorzugsweise mit in der Technik üblichen wäßrig-alkalischenn Entwicklern, die auch kleine Anteile organischer Lösemittel enthalten können, oder aber mit organischen Lösemitteln.

Die bereits im Zusammenhang mit den photopolymerisierbaren Gemischen aufgeführten Träger kommen ebenfalls für positiv arbeitende Kopiermaterialien in Frage, zusätzlich die in der Mikroelektronik üblichen Silizium-, Siliziumdioxid- oder Galliumarsenidoberflächen.

Die Menge der als Säurespender eingesetzten erfindungsgemäßen Verbindungen kann in den positiv arbeitenden Gemischen je nach Substanz und Schicht sehr verschieden sein. Günstigere Ergebnisse werden erhalten zwischen etwa 0,1 und 10 %, bezogen auf den Gesamtfeststoff, bevorzugt sind etwa 0,2 bis 5 %. Für Schichten mit Dicken über 10$\mu$m empfiehlt es sich, relativ wenig Säurespender zu verwenden.

Grundsätzlich ist elektromagnetische Strahlung mit Wellenlängen bis etwa 700 nm zur Belichtung geeignet, Der bevorzugte Wellenlängenbereich erstreckt sich von 300 bis 500 nm. Ihre höchste Empfindlichkeit weisen die erfindungsgemäßen Gemische im Bereich zwischen 350 und 400 nm auf.

Die Vielfalt der erfindungsgemäßen Verbindungen, deren Absorptionsmaxima häufig bei der interessierenden Wellenlänge von 365 nm liegen, gestattet es, den Photoinitiator optimal auf die verwendete Lichtquelle abzustimmen. Als Lichtquellen sind beispielsweise zu nennen: Röhrenlampen, Xenonimpulslampen, metallhalogeniddotierte Quecksilberdampf-Hochdrucklampen und Kohlebogenlampen.

Darüber hinaus ist bei den erfindungsgemäßen lichtempfindlichen Gemischen das Belichten in üblichen Projektions- und Vergrößerungs-Geräten unter dem Licht von Metallfadenlampen und Kontaktbelichtung mit gewöhnlichen Glühbirnen mit Vorteil möglich. Die Belichtung kann auch mit kohärentem Licht eines Lasers erfolgen. Geeignet für die Zwecke vorliegender Erfindung sind beispielsweise Argon-Ionen-Laser, Krypton-Ionen-Laser, Xenon-Ionen-Laser, Farbstoff-Laser, Helium-Cadmium-oder Helium-Neon-Laser. Der Laserstrahl wird im allgemeinen mittels einer vorgegebenen programmierten Strich- und/oder Raster-Bewegung gesteuert.

Das Bestrahlen mit Elektronenstrahlen ist eine weitere Differenzierungsmöglichkeit. Elektronenstrahlen können Gemische, die eine der erfindungsgemäßen Verbindungen und eine durch Säure spaltbare Verbindung enthalten, wie auch viele andere organische Materialien durch-greifend zersetzen und vernetzen, so daß ein negatives Bild entsteht, wenn die unbestrahlten Teile durch Lösemittel oder Belichten ohne Vorlage und Entwickeln entfernt werden.

Bei geringerer Intensität und/oder höherer Schreibgeschwindigkeit des Elektronenstrahls bewirkt dagegen der Elektronenstrahl eine Differenzierung in Richtung höherer Löslichkeit, d. h. die bestrahlten Schichtpartien können vom Entwickler entfernt werden. Die günstigsten Bedingungen können durch Vorversuche leicht ermittelt werden.

Bevorzugte Anwendung finden die die erfindungsgemäßen Verbindungen enthaltenden strahlungsempfindlichen Gemische bei der Herstellung von positiv oder negativ arbeitenden Druckformen, d. h. insbesondere Offset-, Hoch-, Flexo-, autotypischen Tiefdruck- und Siebdruckformen, in Photoresistlösungen und in Trockenresists.

Aufgrund der Eigenschaften der erfindungsgemäßen Verbindungen erhält man dabei lichtempfindliche Aufzeichnungsmaterialien, die gegenüber dem Stand der Technik zahlreiche Vorteile haben. Unter diesen ist besonders die hohe Lichtempfindlichkeit gegenüber im nahen UV-Bereich emittierenden Lichtquellen zu nennen. Dadurch ist die praxisgerechte, rasche Belichtung mit wenig energiereichen, im nahen UV-Bereich emittierenden Lasern gewährleistet. Die häufig bei lichtempfindlichen Gemischen beobachtete hohe Empfindlichkeit gegenüber Luftsauerstoff wird bei Einsatz der erfindungsgemäßen Photoinitiatoren nur in untergeordnetem Maß beobachtet. Ganz besonders vorteilhaft ist jedoch der Umstand, daß die hohe chemische und thermische Stabilität der erfindungsgemäßen Photoinitiatoren eine ungewöhnlich hohe Lagerstabilität der lichtempfindlichen Schicht bewirkt, die eine Vorratshaltung und längere Lagerung der Lösungen bzw. lichtempfindlichen Schichten erlaubt. Schließlich ist als ein weiterer wichtiger Vorteil noch zu vermerken, daß die lichtempfindlichen Gemische bei Verwendung der erfindungsgemäßen Photoinitiatoren bzw. Säurespender praktisch keine katalytisch ausgelösten Dunkelreaktionen auf kritischen Oberflächen, wie z. B. Kupferoberflächen, erleiden, was an sich schon eine Verbesserung gegenüber dem Stand der Technik darstellt. Die geringe Eigenfärbung der erfindungsgemäßen Verbindungen erlaubt ihren praxisgerechten Einsatz auch bei dicken Schichten.

Obwohl die lichtempfindlichen Gemische auch gegenüber sichtbarem Licht empfindlich sind und daher unter geeigneten Bedingungen, z. B. unter Gelblicht, gehandhabt werden müssen, sind die erfindungsgemäßen Verbindungen in kristallinem Zustand ungewöhnlich licht-und thermostabil. Sie lassen sich daher ohne größeren Aufwand unter praktisch normalen Produktionsbedingungen herstellen und handhaben und sind in diesem Zustand lange stabil, so daß eine praxisgerechte Bevorratung mit diesen Verbindungen möglich ist.

Die folgenden Beispiele dienen zur näheren Erläuterung der Erfindung; es erfolgt zunächst die Beschreibung der Herstellung von verschiedenen erfindungsgemäßen Verbindungen, woran sich die Anwendung von einigen dieser Verbindungen in strahlungsempfindlichen Gemischen anschließt.

In den Beispielen stehen Gew.-Teile (Gt) und Vol.-Teile (Vt) im Verhältnis von g zu ml. Prozent- und Mengenangaben sind, wenn nichts anderes angegeben ist, in Gewichtseinheiten zu verstehen.

Herstellungsbeispiel 1

a) 5-Phenyltetrazol

11,5 Gt Benzonitril, 9,2 Gt Lithiumchlorid und 15,7 Gt Natriumazid werden in 400 Vt 2-Methoxy-ethanol suspendiert, und das Gemisch wird 15 Stunden zum Rückfluß erhitzt. Die erkaltete Mischung wird auf 1000 Vt Wasser gegossen und 30 Minuten gerührt. Die unlöslichen Bestandteile werden abfiltriert, und anschließend wird vorsichtig mit konzentrierter Salzsäure versetzt, bis ein pH-Wert von 2 eingestellt ist. Die Mischung wird in einem Eis/Kochsalz-Kältebad auf 0 °C abgekühlt und über Nacht bei dieser Temperatur stehen gelassen. Der Niederschlag wird abgesaugt und über Phosphorpentoxid getrocknet. Ausbeute: 8 Gt 5-Phenyltetrazol.

b) 4-(4,6-Bis-trichlormethyl-s-triazin-2-yl)-benzoesäuremethylester

1. Stufe: Zu 14 Gt Hydroxylammoniumchlorid werden 16 Gt Pyridin gegeben, und die Mischung wird gerührt. Dazu werden unter Rühren 32,8 Gt 4-Methoxycarbonyl-benzaldehyd gegeben, wobei sich die Mischung erwärmt. Nach 10 Minuten wird mit 200 Gt m-Xylol versetzt und das Gemisch an einem Wasserabscheider zum Rückfluß erhitzt. Nach etwa 10 Stunden hat sich die theoretische Menge Wasser abgeschieden. Das Gemisch wird auf Raumtemperatur abkühlen gelassen, mit 200 Gt Diethylether verdünnt und zweimal mit je 150 Gt destilliertem Wasser gewaschen. Die organische Phase wird mit Magnesiumsulfat getrocknet, und die Lösungsmittel werden am Rotationsverdampfer entfernt, wobei letzte Xylolreste unter vermindertem Druck abgezogen werden. Das erhaltene, hauptsächlich aus 4-Cyano-benzoesäuremethylester bestehende Rohprodukt wird aus 150 Gt Ethanol umkristallisiert.

2. Stufe: 16 Gt des vorstehend beschriebenen getrockneten Produkts werden mit 86,6 Gt Trichloracetonitril und 3,2 Gt Aluminiumbromid unter Feuchtigkeitsausschluß gerührt. Die klare Lösung wird auf 24 bis 28 °C temperiert und dann unter Rühren Chlorwasserstoffgas eingeleitet, bis keine HCl-Aufnahme mehr erfolgt (ca. 2 bis 5 Stunden). Das Reaktionsprodukt verfestigt sich in dieser Zeit zusehends. Das Rühren wird eingestellt und die sirupöse Mischung bei Raumtemperatur 24 Stunden nachreagieren gelassen. Das gelbe, feste Reaktionsprodukt wird in 500 Gt Dichlormethan aufgenommen und zweimal mit je 250 Gt destilliertem Wasser gewaschen. Die organische Phase wird über Natriumsulfat getrocknet. Nach Abdampfen des Lösungsmittels wird der weiße Rückstand aus 250 Gt Ethanol umkristallisiert. Ausbeute: 41 Gt = 91 % d. Th. weißer Kristalle von 4-(4,6-Bis-trichlormethyl-s-triazin-2-yl)-benzoesäuremethylester. Smp.: 157 bis 158 °C.

| $C_{13}H_7N_3Cl_6O_2$ (449,9) | | | | |
|---|---|---|---|---|
| ber. | C 34,70 % | H 1,51 % | N 9,34 % | Cl 47,28 % |
| gef. | C 34,6 % | H 1,4 % | N 9,1 % | Cl 47,7 % |

c) 4-(4,6-Bis-trichlormethyl-s-triazin-2-yl)-benzoesüaure

125 Gt 4-(4,6-Bis-trichlormethyl-s-triazin-2-yl)-benzoesäuremethylester, 250 Gt Trichloressigsäure und 2 Gt konz. Schwefelsäure werden unter Rühren auf 175 °C erwärmt. Bei dieser Temperatur wird gebildeter Trichloressigsäuremethylester abdestilliert. Nachdem etwa 40 Gt Trichloressigsäuremethylester abdestilliert sind, wird ein leichtes Vakuum von 270 mbar angelegt, um die Destillation zu vervollständigen. Der sich

verschiedentlich verfestigende Rückstand wird auf 80 °C abkühlen gelassen und dann in 1500 Gt Eiswasser gegeben. Das Gemisch wird 30 Minuten digeriert und anschließend das Produkt abgesaugt. Ausbeute: 87 Gt = 72 % d. Th., weiße Kristalle (aus Toluol) vom Smp 275 °C.

| $C_{12}H_5N_3O_2Cl_6$ (435,9) | | | | |
|---|---|---|---|---|
| ber:<br>gef: | C 33,06<br>C 33,3 | H 1,16<br>H 1,0 | N 9,64<br>N 9,6 | Cl 48,80<br>Cl 48,3 |

d) 4-(4,6-Bis-trichlormethyl-s-triazin-2-yl)-benzoesäurechlorid

Zu 350 Gt Thionylchlorid werden 87 Gt 4-(4,6-Bis-trichlormethyl-s-triazin-2-yl)-benzoesäure gegeben. Beim Erwärmen zum Rückfluß unter Feuchtigkeitsausschluß und unter starkem Rühren ist eine deutliche $SO_2$-Entwicklung zu beobachten. Nach 6 Stunden liegt eine klare Lösung vor. Überschüssiges Thionylchlorid wird, zuletzt unter Anlegen von Vakuum, abdestilliert. Der Rückstand, der in praktisch quantitativer Ausbeute reines 4-(4,6-Bis-trichlormethyl-s-triazin-2-yl)-benzoylchlorid enthält, wird aus Hexan umkristallisiert, Smp. 100 bis 101 °C.

| $C_{12}H_4N_3OCl_7$ (454,3) | | | | |
|---|---|---|---|---|
| ber:<br>gef: | C 31,72<br>C 31,9 | H 0,89<br>H 0,7 | N 9,25<br>N 9,3 | Cl 54,62<br>Cl 54,7 |

e) 2-[4-(4,6-Bis-trichlormethyl-s-triazin-2-yl)phenyl]-5-phenyl-1,3,4-oxadiazol (Verbindung Nr. 1)

5 Gt 5-Phenyltetrazol und 15,5 Gt 4-(4,6-Bis-trichlormethyl-s-triazin-2-yl)-benzoylchlorid werden in 125 Vt Pyridin gelöst und langsam zum Rückfluß erhitzt. Bei etwa 80 °C beginnt eine Stickstoffentwicklung, die nach etwa 1 Stunde beendet ist. Das abgekühlte Gemisch wird auf 500 Vt Eiswasser gegossen, der ausfallende Niederschlag wird abfiltriert und nach Trocknung aus 2-Methoxy-ethanol umkristallisiert. Ausbeute: 15,2 Gt = 83,5 % d. Th., fleischfarbene Kristalle, Smp. 226 bis 227,5 °C.

| $C_{19}H_9Cl_6N_5O$ (536,0) | | | | |
|---|---|---|---|---|
| ber:<br>gef: | C 42,57<br>C 42,3 | H 1,69<br>H 1,6 | N 13,06<br>N 12,8 | Cl 39,68<br>Cl 40,1 |

UV ($CH_2Cl_2$): $\lambda_{max}$ = 330 nm.

Herstellungsbeispiel 2:

a) 5-(3,4-Dimethoxyphenyl)-tetrazol

18,1 Gt 3,4-Dimethoxybenzonitril, 9,1 Gt Lithiumchlorid und 15,7 Gt Natriumazid werden in 400 Vt 2-Methoxyethanol 16 Stunden zum Rückfluß erhitzt. Nach dem Abkühlen werden etwa 2/3 des Lösungsmittels abdestilliert, und die restliche Mischung wird in 1000 Vt Wasser eingegossen. Die erhaltene klare Lösung wird auf 0 °C gekühlt, angesäuert und der ausfallende Niederschlag abfiltriert, mit Wasser gewaschen und getrocknet. Ausbeute: 13,5 Gt 5-(3,4-Dimethoxyphenyl)-tetrazol.

b) 2-[4-(4,6-Bis-trichlormethyl-s-triazin-2-yl)-phenyl]-5-(3,4-dimethoxyphenyl)-1,3,4-oxadiazol (Verbindung Nr. 7)

20 Gt 5-(3,4-Dimethoxyphenyl)-tetrazol und 44 Gt 4-(4,6-Bis-trichlormethyl-s-triazin-2-yl)benzoylchlorid werden in 200 Vt Pyridin langsam zum Rückfluß erhitzt, wobei eine starke Stickstoffentwicklung auftritt. Nach 1,5 Stunden wird abgekühlt, die Lösung in 1000 Vt Wasser gegossen, und nach vervollständigter Fällung wird das Produkt abgesaugt. Nach Trocknung wird aus 2-Methoxyethanol umkristallisiert. Ausbeute:

36 Gt = 62,9 % d. Th., gelbliche Kristalle, Smp. 214 bis 215 °C.

| $C_{21}H_{13}Cl_6N_5O_3$ (596,1) | | | | |
|---|---|---|---|---|
| ber: | C 42,31 | H 2,20 | N 11,75 | Cl 35,69 |
| gef: | C 42,1 | H 2,2 | N 11,7 | Cl 36,2 |

UV ($CH_2Cl_2$): $\lambda_{max}$ = 353 nm.

Herstellungsbeispiel 3:

a) 3-(4,6-Bis-trichlormethyl-s-triazin-2-yl)benzoylchlorid

240 Gt Thionylchlorid und 240 Gt Toluol werden vermischt und mit 140 Gt 3-Cyano-benzoesäure versetzt. Die Suspension wird unter Feuchtigkeitsausschluß gerührt und zum Rückfluß erhitzt. Nach etwa 5 Stunden ist die $SO_2$-Entwicklung beendet und die Lösung klar. Überschüssiges Thionylchlorid und Toluol werden abdestilliert und der Rückstand vorsichtig in 600 Gt Methanol gegossen. Das Gemisch wird 24 Stunden stehen gelassen und dann zur Vervollständigung der Fällung auf 0 °C gekühlt. Der Niederschlag aus 3-Cyano-benzoesäuremethylester wird abgesaugt und über Phosphorpentoxid getrocknet.

Die vorstehend beschriebene Verbindung wird analog wie im Herstellungsbeispiel 1 beschrieben mit Trichloracetonitril zum 3-(4,6-Bis-trichlormethyl-s-triazin2-yl)-benzoesäuremethylester umgesetzt (Smp.: 115 bis 117 ° C.). Aus diesem wird, in gleicher Weise wie dort, durch Umsetzung mit Trichloressigsäure die entsprechende Benzoesäure (Smp.: 211,5 °C) und daraus durch Umsetzen mit Thionylchlorid das Säurechlorid (Smp.: 102 bis 103 °C) hergestellt.

b) 2-[3-(4,6-Bis-trichlormethyl-s-triazin-2-yl)-phenyl]-5-(3,4-dimethoxyphenyl)-1,3,4-oxadiazol (Verbindung Nr. 23)

20 Gt 5-(3,4-Dimethoxyphenyl)-tetrazol werden mit 44 Gt 3-(4,6-Bis-trichlormethyl-s-triazin-2-yl)-benzoesäurechlorid in 400 Vt Pyridin erhitzt. Die weitere Aufarbeitung erfolgt wie vorstehend angegeben. Ausbeute: 52 Gt = 90,8 % d. Th., praktisch farblose Kristalle, Smp. 195 bis 196 °C.

| $C_{21}H_{13}Cl_6N_5O_3$ (596,1) | | | | |
|---|---|---|---|---|
| ber: | C 42,31 | H 2,20 | N 11,75 | Cl 35,69 |
| gef: | C 42,2 | H 2,2 | N 11,7 | Cl 36,9 |

UV ($CH_2Cl_2$): $\lambda_{max}$ = 306 nm.

Herstellungsbeispiel 4

a) 4-(4,6-Bis-trichlormethyl-s-triazin-2-yl)zimtsäuremethylester

Stufe 1: 50 Gt 4-Cyanozimtsäure, 27,7 Gt absolutes Methanol, 200 Gt 1,2-Dichlorethan und 1,6 Gt Toluolsulfonsäure werden ca. 15 Stunden zum Rückfluß erhitzt. Die Kontrolle durch Dünnschichtchromatographie zu diesem Zeitpunkt zeigt, daß die Umsetzung vollständig abgelaufen ist. Die klare Lösung wird mit Eis gekühlt, worauf ein Teil des Produkts auskristallisiert und durch Filtration isoliert wird. Die Mutterlauge wird mit 5 %iger Natriumhydrogencarbonatlösung und zweimal mit Wasser gewaschen. Nach dem Trocknen der organischen Phase mit Magnesiumsulfat wird das Lösungsmittel abgezogen und das erhaltene Produkt über Phosphorpentoxid getrocknet. Die beiden Chargen von 4-Cyano-zimtsäuremethylester werden vereinigt, da beide gleiche Reinheit aufweisen.

Aus dem erhaltenen Ester wird analog Herstellungsbeispiel 1 (b) der 4-(4,6-Bis-trichlormethyl-s-triazin-2-yl)-zimtsäuremethylester hergestellt.

b) 4-(4,6-Bis-trichlormethyl-s-triazin-2-yl)-zimtsäure

In einen mit trockenem Stickstoff gespülten Kolben werden 450 Gt trockenes 1,2-Dichlorethan, 49,2 Gt Hexamethyldisilan und 85,3 Gt Jod vorgelegt. Dazu werden portionsweise 80 Gt 4-(4,6-Bis-trichlormethyl-s-triazin-2-yl)-zimtsäuremethylester gegeben, wobei die gelegentlich exotherm ablaufende Reaktion durch Kühlung unter Kontrolle gehalten wird. Das Gemisch wird nach vollständiger Zugabe und unter Verwendung eines bei -20 °C arbeitenden Kühlersystems zum Rückfluß erhitzt und solange erwärmt, bis die Dünnschichtchromatographie eine praktisch vollständige Umsetzung anzeigt (4 bis 12 Stunden). Nach dem Erkalten wird das Reaktionsgemisch mit 400 Gt Wasser versetzt und am Rotationsverdampfer eingeengt, bis das Dichlorethan abgedampft ist. Die zurückbleibende wäßrige Mischung wird mit Methanol versetzt, wobei die Säure quantitativ ausfällt. Diese wird abgesaugt und aus Eisessig (umkristallisiertes Produkt enthält dann 1 Molekül Eisessig) oder einer Wasser/Eisessig-Mischung umkristallisiert. Ausbeute: 60,5 Gt = 78 % d. Th., Smp. 233 bis 234 °C

| $C_{14}H_7N_3O_2Cl_6$ (461,9) | | | | |
|---|---|---|---|---|
| ber: | C 36,40 | H 1,53 | N 9,10 | Cl 46,05 |
| gef: | C 36,2 | H 1,35 | N 8,9 | Cl 46,5 |

Aus der erhaltenen Verbindung wird analog Herstellungsbeispiel 1(d) das 4-(4,6-Bis-trichlormethyl-s-triazin-2-yl)-cinnamoylchloridhergestellt (Smp.: 156 bis 158 °C).

c) 2-[4-(4,6-Bis-trichlormethyl-s-triazin-2-yl)-phenyl-ethenyl]-5-(3,4-dimethoxyphenyl)-1,3,4-oxadiazol (Verbindung Nr. 37)

20 Gt 5-(3,4-Dimethoxyphenyl)tetrazol werden mit 48 Gt 4-(4,6-Bis-trichlormethyl-s-triazin-2-yl)-zimtsäurechlorid in 400 Vt Pyridin zum Rückfluß erhitzt. Nach etwa einstündiger Reaktionszeit wird das dunkle Gemisch abgekühlt und auf Wasser gegossen. Durch langsame Zugabe von Salzsäure wird ein pH-Wert von 3 bis 4 eingestellt und das Gemisch mit einer 1 : 1-Mischung aus Ether und Tetrahydrofuran ausgeschüttelt. Die organische Phase wird mit Wasser, verdünnter Natriumhydrogencarbonat-Lösung, erneut mit Wasser gewaschen und über Calciumchlorid getrocknet. Die Lösungsmittel werden abgedampft und der Rückstand in Toluol digeriert. Die nicht in Lösung gegangenen Bestandteile werden abfiltriert. Ausbeute: 30 Gt = 52,4 % d. Th., gelbe Kristalle, Smp. 241,5 bis 242 °C (Zersetzung).

| $C_{23}H_{15}Cl_6N_5O_3$ (622,1) | | | | |
|---|---|---|---|---|
| ber: | C 44,40 | H 2,43 | N 11,26 | Cl 34,19 |
| gef: | C 44,7 | H 2,5 | N 11,1 | Cl 34,4 |

UV ($CH_2Cl_2$): $\lambda_{max}$ = 369 nm.

Herstellungsbeispiel 5:

a) 5-(3,4-Dimethoxy-phenylethenyl)-tetrazol

9,8 Gt 3,4-Dimethoxyzimtsäurenitril, 4,3 Gt Lithiumchlorid und 7,4 Gt Natriumazid werden in 200 Vt 2-Methoxy-ethanol 16 Stunden zum Rückfluß erhitzt. Nach dem Abkühlen wird die Mischung in 4000 Vt Wasser eingegossen. Die erhaltene klare Lösung wird auf 0 °C gekühlt, angesäuert und der ausfallende Niederschlag abfiltriert, mit Wasser gewaschen und getrocknet. Ausbeute: 9 Gt 5-(3,4-Dimethoxy-phenylethenyl)-tetrazol.

b) 2-[4-(4,6-Bis-trichlormethyl-s-triazin-2-yl)-phenyl]-5-(3,4-dimethoxy-phenylethenyl)-1,3,4-oxadiazol (Verbindung Nr. 30)

20 Gt der unter (a) beschriebenen Verbindung und 40 Gt 4-(4,6-Bis-trichlormethyl-s-triazin-2-yl)-zimtsäurechlorid werden in 200 Vt Pyridin langsam zum Rückfluß erhitzt, wobei eine starke Stickstoffentwicklung auftritt. Nach 1,5 Stunden wird abgekühlt, die Lösung in 500 Vt Wasser gegossen, und nach

vervollständigter Fällung wird das Produkt abgesaugt. Nach dem Trocknen wird aus 2-Methoxy-ethanol umkristallisiert. Ausbeute: 28 Gt = 52,4 % d. Th., gelbliche Kristalle, Smp. 207 bis 208 °C.

| $C_{23}H_{15}Cl_6N_5O_3$ (622,1) | | | | |
|---|---|---|---|---|
| ber: | C 44,40 | H 2,43 | N 11,26 | Cl 34,19 |
| gef: | C 44,4 | H 2,4 | N 11,2 | Cl 34,4 |

UV ($CH_2Cl_2$): $\lambda_{max}$ = 373 nm.

Herstellungsbeispiel 6

a) 5-(1-Naphthyl)-tetrazol

40 Gt 1-Naphthonitril, 21,5 Gt Lithiumchlorid und 34,7 Gt Natriumazid werden in 750 Vt 2-Methoxy-ethanol 16 Stunden zum Rückfluß erhitzt. Nach dem Abkühlen wird die Mischung in 4000 Vt Wasser eingegossen. Die erhaltene Mischung wird filtriert, auf 0 °C gekühlt und der nach Ansäuern ausfallende Niederschlag abfiltriert, mit Wasser gewaschen und getrocknet. Ausbeute: 29 Gt 5-(1-Naphthyl)-tetrazol.

b) 2-[4-(4,6-Bis-trichlormethyl-s-triazin-2-yl)-phenyl]-5-(1-naphthyl)-1,3,4-oxadiazol (Verbindung Nr. 16)

26,7 Gt der unter (a) beschriebenen Verbindung und 93 Gt 4-(4,6-Bis-trichlormethyl-s-triazin-2-yl)-benzoylchlorid werden in 300 Vt Pyridin langsam zum Rückfluß erhitzt, wobei eine starke Stickstoffentwicklung auftritt. Nach 1,5 Stunden wird abgekühlt, die Lösung in 800 Vt Wasser gegossen und nach vervollständigter Fällung wird das Produkt abgesaugt. Nach Trocknung wird aus 2-Methoxy-ethanol umkristallisiert. Ausbeute: 57 Gt = 71,5 % d. Th., gelbliche Kristalle, Smp. 298 °C.

| $C_{23}H_{11}Cl_6N_5O$ (586,1) | | | | |
|---|---|---|---|---|
| ber: | C 47,13 | H 1,89 | N 11,95 | Cl 36,29 |
| gef: | C 47,1 | H 1,9 | N 11,7 | Cl 36,0 |

UV ($CH_2Cl_2$): $\lambda_{max}$ = 348 nm.

Herstellungsbeispiel 7

a) 5-(2-Naphthyl)-tetrazol

40 Gt 2-Naphthonitril, 21,5 Gt Lithiumchlorid und 34,7 Gt Natriumazid werden in 750 Vt 2-Methoxy-ethanol 16 Stunden zum Rückfluß erhitzt. Nach dem Abkühlen wird die Mischung in 4000 Vt Wasser eingegossen. Die erhaltene Mischung wird filtriert, auf 0 °C gekühlt und angesäuert. Der ausfallende Niederschlag wird abfiltriert, mit Wasser gewaschen und getrocknet. Ausbeute: 46 Gt 5-(2-Naphthyl)-tetrazol.

b) 2-[4-(4,6-Bis-trichlormethyl-s-triazin-2-yl)-phenyl]-5-(2-naphthyl)-1,3,4-oxadiazol (Verbindung Nr. 17)

19,6 Gt der unter (a) beschriebenen Verbindung und 45,4 Gt 4-(4,6-Bis-trichlormethyl-s-triazin-2-yl)-benzoylchlorid werden in 300 Vt Pyridin langsam zum Rückfluß erhitzt, wobei eine starke Stickstoffentwicklung auftritt. Nach 1,5 Stunden wird abgekühlt, die Lösung in 800 Vt Wasser gegossen, und nach vervollständigter Fällung wird das Produkt abgesaugt. Nach Trocknung wird aus Ethanol umkristallisiert. Ausbeute: 46 Gt = 77,8 % d. Th., schwach gelbliche Kristalle, Smp. 248 bis 250 °C.

| $C_{23}H_{11}Cl_6N_5O$ (586,1) | | | | |
|---|---|---|---|---|
| ber: | C 47,13 | H 1,89 | N 11,95 | Cl 36,29 |
| gef: | C 46,8 | H 1,7 | N 11,6 | Cl 36,6 |

UV ($CH_2Cl_2$): $\lambda_{max}$ = 336 nm.

Herstellungsbeispiel 8

a) 5-(4-Diphenylaminophenyl)-tetrazol

81 Gt 4-Diphenylamino-benzonitril, 47 Gt Lithiumchlorid und 72 Gt Natriumazid werden in 1050 Vt 2-Methoxyethanol 96 Stunden zum Rückfluß erhitzt. Nach dem Abkühlen wird die Mischung in 3000 Vt Wasser eingegossen. Die erhaltene Mischung wird mit Aktivkohle versetzt, filtriert, auf 0 °C gekühlt und auf pH = 5 angesäuert. Der ausfallende Niederschlag wird abfiltriert, mit Wasser gewaschen und getrocknet. Ausbeute: 84 Gt 5-(4-Diphenylamino-phenyl)-tetrazol.

b) 2-[3-(4,6-Bis-trichlormethyl-s-triazin-2-yl)-phenyl]-5-(4-diphenylaminophenyl)-1,3,4-oxadiazol (Verbindung Nr. 26)

31,4 Gt der unter (a) beschriebenen Verbindung und 45,4 Gt 3-(4,6-Bis-trichlormethyl-s-triazin-2-yl)-benzoylchlorid werden in 300 Vt Pyridin langsam zum Rückfluß erhitzt, wobei eine starke Stickstoffentwicklung auftritt. Nach 1,5 Stunden wird abgekühlt, die Lösung in 800 Vt Wasser gegossen, und nach vervollständigter Fällung wird das Produkt abgesaugt. Nach dem Trocknen wird aus Ethanol/Toluol umkristallisiert. Ausbeute: 52 Gt = 73,9 % d. Th., gelbe Kristalle, Smp. 238 bis 239 °C.

| $C_{31}H_{18}Cl_6N_6O$ (703,2) | | | | |
|---|---|---|---|---|
| ber: | C 52,96 | H 2,58 | N 11,95 | Cl 30,25 |
| gef: | C 52,5 | H 2,8 | N 11,6 | Cl 30,3 |

UV ($CH_2Cl_2$): $\lambda_{max}$ = 360 nm.

Herstellungsbeispiel 9

2-[4-(4,6-Bis-trichlormethyl-s-triazin-2-yl)-phenyl]-5-(4-diphenylamino-phenyl)-1,3,4-oxadiazol (Verbindung Nr. 18)

31,4 Gt der im Herstellungsbeispiel 8 unter (a) beschriebenen Verbindung und 45,4 Gt 4-(4,6-Bis-trichlormethyl-s-triazin-2-yl)-benzoylchlorid werden in 300 Vt Pyridin langsam zum Rückfluß erhitzt, wobei eine starke Stickstoffentwicklung auftritt. Nach 1,5 Stunden wird abgekühlt, die Lösung in 800 Vt Wasser gegossen, und nach vervollständigter Fällung wird das Produkt abgesaugt. Nach dem Trocknen wird aus Ethanol/Chloroform umkristallisiert. Ausbeute: 46 Gt = 65,4 % d. Th., gelbe Kristalle, Smp. 209,5 bis 210,5 °C.

| $C_{31}H_{18}Cl_6N_6O$ (703,2) | | | | |
|---|---|---|---|---|
| ber: | C 52,95 | H 2,58 | N 11,95 | Cl 30,25 |
| gef: | C 53,1 | H 2,4 | N 11,5 | Cl 29,4 |

UV ($CH_2Cl_2$): $\lambda_{max}$ = 403 nm.

Herstellungsbeispiel 10

a) 5-(4-Chlorphenyl)-tetrazol

15,3 Gt 4-Chlorbenzonitril, 9,1 Gt Lithiumchlorid und 15,7 Gt Natriumazid werden in 450 Vt 2-Methoxyethanol 24 Stunden zum Rückfluß erhitzt. Nach dem Abkühlen wird die Mischung in 1000 Vt Wasser eingegossen. Die erhaltene Mischung wird filtriert, auf 0 °C gekühlt und auf pH = 3 angesäuert. Der ausfallende Niederschlag wird abfiltriert, mit Wasser gewaschen und getrocknet. Ausbeute: 18 Gt 5-(4-Chlorphenyl)-tetrazol.

b) 2-[4-(4,6-Bis-trichlormethyl-s-triazin-2-yl)-phenyl]-5-(4-chlor-phenyl)-1,3,4-oxadiazol (Verbindung Nr. 9)

24,6 Gt der unter (a) beschriebenen Verbindung und 93,0 Gt 4-(4,6-Bis-trichlormethyl-s-triazin-2-yl)-benzoylchlorid werden in 300 Vt Pyridin langsam zum Rückfluß erhitzt, wobei eine starke Stickstoffentwicklung auftritt. Dabei bildet sich zu keinem Zeitpunkt der Reaktion eine klare Lösung. Nach 1,5 Stunden wird abgekühlt, die Lösung in 800 Vt Wasser gegossen, und nach vervollständigter Fällung wird das Produkt abgesaugt. Nach dem Trocknen wird aus 2-Methoxy-ethanol umkristallisiert. Ausbeute: 70 Gt = 90,2 % d. Th., fleischfarbene, leicht gelbe Kristalle, Smp. 273 °C.

| $C_{19}H_8Cl_7N_5O$ (570,5) | | | | |
|---|---|---|---|---|
| ber: | C 40,00 | H 1,41 | N 12,28 | Cl 43,50 |
| gef: | C 39,8 | H 1,4 | N 12,1 | Cl 43,4 |

UV ($CH_2Cl_2$): $\lambda_{max}$ = 332 nm.

Herstellungsbeispiel 11

Die Stufe (a) des Herstellungsbeispiels 1 wird derart abgewandelt, daß das Lithiumchlorid durch eine äquivalente Menge Ammoniumchlorid ersetzt und anstelle von 2-Methoxy-ethanol Dimethylformamid verwendet wird. Die Mischung wird 16 Stunden auf 110 °C erwärmt, wobei im Kühler größere Kristalle von Ammoniumazid auskristallisieren. Die Aufarbeitung erfolgt wie im Herstellungsbeispiel 1 angegeben. Man erhält eine Ausbeute von 9 Gt 5-Phenyltetrazol.

Die weitere Umsetzung erfolgt wie im Herstellungsbeispiel 1 angegeben; das erhaltene Produkt weist einen Schmelzpunkt von 227 ° C auf.

Herstellungsbeispiel 12

Die Stufe (a) des Herstellungsbeispiels 1 wird derart abgewandelt, daß das Lithiumchlorid durch eine äquivalente Menge Ammoniumchlorid ersetzt wird. Die Mischung wird 16 Stunden zum Rückfluß erhitzt, wobei im Kühler Ammoniumazid auskristallisiert. Die Aufarbeitung erfolgt wie im Herstellungsbeispiel 1 angegeben. Man erhält eine Ausbeute von 5 Gt 5-Phenyltetrazol.

In der folgenden Tabelle sind alle hergestellten und untersuchten Verbindungen der Formel I zusammengestellt:

Tabelle I
Verbindungen der Formel I

| Verb.Nr. | $R^1$ | n | m | $R^2$ | $R^3$ |
|---|---|---|---|---|---|
| 1 | Phenyl | 0 | 0 | H | X |
| 2 | 4-Tolyl | " | " | " | " |
| 3 | 3-Tolyl | " | " | " | " |
| 4 | 4-Methoxyphenyl | " | " | " | " |
| 5 | 3-Methoxyphenyl | " | " | " | " |
| 6 | 2-Methoxyphenyl | " | " | " | " |
| 7 | 3,4-Dimethoxyphenyl | " | " | " | " |
| 8 | 3,4,5-Trimethoxyphenyl | " | " | " | " |
| 9 | 4-Chlorphenyl | " | " | " | " |
| 10 | 2,4-Dichlorphenyl | " | " | " | " |
| 11 | 4-Nitrophenyl | " | " | " | " |
| 12 | 4-Cyanophenyl | " | " | " | " |
| 13 | 4-Formylphenyl | " | " | " | " |
| 14 | 3-Trifluormethylphenyl | " | " | " | " |
| 15 | Biphenyl-4-yl | " | " | " | " |
| 16 | 1-Naphthyl | " | " | " | " |
| 17 | 2-Naphthyl | " | " | " | " |
| 18 | 4-Diphenylamino-phenyl | " | " | " | " |
| 19 | 3-Pyridyl | " | " | " | " |
| 20 | N-Ethyl-carbazol-3-yl | " | " | " | " |
| 21 | 4-Tolyl | " | " | X | H |
| 22 | 4-Methoxyphenyl | " | " | " | " |
| 23 | 3,4-Dimethoxyphenyl | " | " | " | " |
| 24 | 4-Chlorphenyl | " | " | " | " |
| 25 | Biphenyl-4-yl | " | " | " | " |

Tabelle I (Verbindungen der Formel I) - Fortsetzung

| Verb.Nr. | $R^1$ | n | m | $R^2$ | $R^3$ |
|---|---|---|---|---|---|
| 26 | 4-Diphenylamino-phenyl | 0 | 0 | X | H |
| 27 | N-Ethyl-carbazol-3-yl | " | " | " | " |
| 28 | Phenyl | 1 | " | H | X |
| 29 | 4-Methoxy-phenyl | " | " | " | " |
| 30 | 3,4-Dimethoxy-phenyl | " | " | " | " |
| 31 | 3,4-Methylendioxy-phenyl | " | " | " | " |
| 32 | Phenyl | " | " | X | H |
| 33 | 4-Tolyl | " | " | " | " |
| 34 | 4-Methoxyphenyl | " | " | " | " |
| 35 | 3,4-Dimethoxy-phenyl | " | " | " | " |
| 36 | 4-Tolyl | 0 | 1 | H | X |
| 37 | 3,4-Dimethoxy-phenyl | " | " | " | " |
| 38 | 4-Methoxyphenyl | 1 | " | " | " |
| 39 | 4-Tolyl | 0 | " | X | H |
| 40 | Biphenyl-4-yl | " | " | " | " |
| 41 | 4-Tolyl | 1 | " | " | " |
| 42 | 4-Methoxyphenyl | " | " | " | " |

X = 4,6-Bis-trichlormethyl-s-triazin-2-yl

Die nicht in den Herstellungsbeispielen beschriebenen Verbindungen weisen Absorptionsmaxima bei den in der folgenden Tabelle II angegebenen Wellenlängen auf (Lösungsmittel $CH_2Cl_2$):

Tabelle II

| Nr. | λ max (nm) | Nr. | λ max (nm) |
|---|---|---|---|
| 2 | 336 | 24 | 304 |
| 3 | 332 | 25 | 323 |
| 4 | 348 | 27 | 379 |
| 5 | 338 | 28 | 347 |
| 6 | 351 | 29 | 368 |
| 8 | 354 | 31 | 370 |
| 10 | 333 | 32 | 318 |
| 11 | 332 | 33 | 321 |
| 12 | 330 | 34 | 333 |
| 13 | 332 | 35 | 334 |
| 14 | 325 | 36 | 361 |
| 15 | 342 | 38 | 377 |
| 19 | 335 | 39 | 320 |
| 20 | 426 | 40 | 323 |
| 21 | 304 | 41 | 339 |
| 22 | 310 | 42 | 351 |

Anwendungsbeispiel 1

Eine elektrochemisch aufgerauhte und anodisierte Aluminiumplatte mit einer Oxidschicht von 2,5 g/m$^2$ wurde mit einer wäßrigen Lösung von Polyvinylphosphonsäure vorbehandelt. Das Trägermaterial wurde mit einer Lösung nachstehender Zusammensetzung überzogen:

91,2 Gt einer 31 %igen Lösung eines Terpolymerisats aus Styrol, n-Hexylmethacrylat und Methacrylsäure (10:60:30) mit der Säurezahl 190 in Butanon,

54,9 Gt einer 51,5 %igen Lösung des Umsetzungsprodukts aus 1 mol Hexamethylendiisocyanat und 2 mol Hydroxyethylmethacrylat,

2,8 Gt der Verbindung Nr. 4 und

660 Gt 2-Methoxy-ethanol

Es wurde durch Aufschleudern und 2 Minuten Trocknen bei 100 °C ein Trockenschichtgewicht von 3,0 g/m$^2$ erhalten. Die lichtempfindliche Schicht wurde mit einer Polyvinylalkoholdeckschicht überzogen.

Die erhaltene Druckplatte wurde mittels einer 5 kW-Metallhalogenidlampe im Abstand von 110 cm unter einem 13-stufigen Belichtungskeil mit zusätzlichen Strich-und Rasterelementen 5 Sekunden belichtet. Nach dieser Belichtung wurde die Platte eine Minute auf 100 °C erwärmt.

Anschließend wurde mit einem Entwickler nachstehender Zusammensetzung entwickelt:

60 Gt Natriummetasilikat x 9 $H_2O$,

1,06 Gt Strontiumchlorid x 6 $H_2O$,

0,6 Gt nichtionogenes Netzmittel und

2000 Gt vollentsalztes Wasser.

Die Platte war bis zur Stufe 4 vernetzt. Die Wiedergabe der feinen Strich- und Rasterelemente war in zufriedenstellender Weise gegeben. Die Platte nahm beim Einspannen in eine Bogenoffsetmaschine die angebotene Farbe willig an und ergab eine Druckauflage von mehr als 100.000.

Anwendungsbeispiel 2

Es wurde eine Beschichtungslösung nachstehender Zusammensetzung bereitet und auf eine wie im Anwendungsbeispiel 1 beschrieben vorbehandelte Aluminiumplatte zu einem Trockenschichtgewicht von 2,8 g/m$^2$ aufgetragen:

102,6 Gt eines Copolymeren aus Methylmethacrylat und Methacrylsäure (82:18) mit der Säurezahl 118, zugesetzt als 34,4 %ige Lösung in Butanon,

36 Gt Trimethylolethantriacrylat,

0,7 Gt eines blauen Azofarbstoffs, erhalten durch Kuppeln von 2,4-Dinitro-6-chlor-benzol-diazoniumsalz mit 2-Methoxy-5-acetylamino-N-cyanoethyl-N-hydroxyethyl-anilin und

1,56 Gt der Verbindung Nr. 7 in

462 Gt 2-Methoxy-ethanol
Die lichtempfindliche Schicht wurde mit einer Deckschicht aus Polyvinylalkohol überzogen, 30 Sekunden wie im Anwendungsbeispiel 1 beschrieben belichtet und ohne zusätzliches Erwärmen mit dem dort angegebenen Entwickler entwickelt.

Man erhielt eine Platte mit hoher Auflösung, die beim Einspannen in eine Bogenoffsetmaschine etwa 200.000 Drucke lieferte.

Anwendungsbeispiel 3

Wie im Anwendungsbeispiel 1 beschrieben, wurde eine lichtempfindliche Lösung nachstehender Zusammensetzung auf ein Aluminiumblech zu einem Trockenschichtgewicht von 3,0 g/m$^2$ aufgebracht und mit einer Deckschicht überzogen:

32,83 Gt eines Umsetzungsprodukts aus einem Polyvinylbutyral mit 71 Gew.-% Vinylbutyral-, 2 Gew.-% Vinylacetat- und 27 Gew.-% Vinylalkoholeinheiten und Propenylsulfonylisocyanat mit der Säurezahl 145, zugesetzt als 12 %ige Lösung in Tetrahydrofuran,
0,03 Gt des im Anwendungsbeispiel 2 angegebenen blauen Azofarbstoffs,
3,94 Gt des im Anwendungsbeispiel 1 beschriebenen Monomeren und
0,37 Gt der Verbindung Nr. 16 in
87,42 Gt 2-Methoxy-ethanol
Belichtung und Entwicklung erfolgten wie im Anwendungsbeispiel 1 angegeben. Hier wurde bei einer Belichtungszeit von 12 Sekunden eine vollvernetzte Stufe 5 beobachtet; alle Raster- und Strichelemente waren in zufriedenstellender Weise wiedergegeben. Beim Druck wurden 160.000 Bögen erzielt.

Anwendungsbeispiel 4

Eine Lösung von
66 Gt des im Anwendungsbeispiel 1 beschriebenen Terpolymerisats,
42 Gt Polypropylenglykol-420-dimethacrylat,
0,2 Gt des im Anwendungsbeispiel 2 angegebenen Farbstoffs,
2,5 Gt der Verbindung Nr. 26 in
240 Gt Butanon und
30 Gt 2-Methoxy-ethanol
wurden auf eine mit 35 µm dicker Kupferfolie kaschierte Phenoplast-Schichtstoffplatte so aufgeschleudert, daß nach dem Trocknen bei 100 °C eine Schichtdicke von 45 µm erhalten wurde. Die Platte wurde mittels einer 5 kW-Metallhalogenidlampe im Abstand von 110 cm zwischen Lampe und Vakuumrahmen 40 Sekunden belichtet. Als Vorlage diente sowohl ein 13-stufiger Belichtungskeil mit Dichteinkrementen von 0,15 als auch eine Strichvorlage mit Linienbreiten und Abständen bis herab zu 80 µm.

Nach der Belichtung wurde die Schicht mit 0,8 %iger Natriumcarbonatlösung in einem Sprühentwicklungsgerät 100 Sekunden lang entwickelt. Es wurden 5 vollvernetzte Keilstufen erhalten.

Die Platte wurde dann 30 Sekunden mit Leitungswasser gespült, 30 Sekunden mit einer 15 %igen Ammoniumperoxydisulfat-Lösung geätzt, erneut mit Wasser gespült, 30 Sekunden in 10 %ige Schwefelsäure getaucht und sodann nacheinander in den folgenden Elektrolytbädern galvanisiert:

1) 50 Minuten in einem Kupferelektrolytbad der Firma Schlötter, Geislingen/Steige
Typ "Glanzkupferbad PC".
Stromdichte: 2,5 A/dm$^2$
Metallaufbau: ca. 25 µm
Temperatur: Raumtemperatur

2) 15 Minuten in einem Bleizinnbad LA der Firma Schlötter, Geislingen/Steige
Stromdichte: 2 A/dm$^2$
Metallaufbau: 15 µm
Temperatur: Raumtemperatur

Die Platte zeigte keine Unterwanderungen oder Beschädigungen. Der Überhang bzw. die Neigung einer Flanke der Resistschicht betrug weniger als 10 µm bei einer Resistbahnbreite von 140 µm.

Die Resistschablone konnte dann in 5 %iger KOH-Lösung bei 50 °C entfernt und das freigelegte Kupfer in den üblichen Ätzmedien weggeätzt werden.

Anwendungsbeispiel 5

Auf eine mechanisch aufgerauhte Aluminiumplatte wurde eine Lösung aus
75 Gt eines Kresol-Formaldehyd-Novolaks mit einem Schmelzbereich von 105 - 120 °C,
23,8 Gt eines Polyacetals aus Triethylenglykol und 2-Butyraldehyd,
0,02 Gt Kristallviolettbase und
0,6 Gt der Verbindung Nr. 36 in
24 Gt 2-Methoxy-ethanol und
275 Gt Butanon
aufgeschleudert und getrocknet. Es wurde durch eine Vorlage belichtet, die einen Stufenkeil und feine Strich- und Rasterelemente aufwies. Die Entwicklung erfolgte mit einer Lösung aus
5,5 Gt Natriummetasilikat x 9 $H_2O$,
3,4 Gt Trinatriumphosphat x 12 $H_2O$ und
0,4 Gt Natriumdihydrogenphosphat in
90,7 Gt entsalztem Wasser.

Bei einer Belichtungszeit von 35 Sekunden und Entwickeln nach 10 Minuten Wartezeit wurden 4 vollentwickelte Keilstufen erhalten. Die Wiedergabe der Testelemente erfolgte bis herab in den 10 µm-Bereich.

Anwendungsbeispiel 6

Es wurde eine Positiv-Trockenresistlösung nachstehender Zusammensetzung hergestellt:
21,2 Gt des im Anwendungsbeispiel 5 beschriebenen Novolaks,
10 Gt des Bis-(5-ethyl-5-butyl-1,3-dioxan-2-yl)-ethers von 2-Ethyl-2-butyl-1,3-propandiol,
0,05 Gt Kristallviolettbase,
3,8 Gt Polyethylacrylat, niedrigviskos und
0,25 Gt der Verbindung Nr. 42 in
65 Gt Butanon.

Damit wurde eine mit wäßriger Trichloressigsäure/Polyvinylalkohol-Lösung vorbehandelte biaxial verstreckte und thermofixierte 25 µm dicke Polyesterfolie beschichtet. Das Trockenschichtgewicht betrug 45 $g/m^2$. Diese Schicht wurde beidseitig auf ein Kupferblech laminiert, und nach dem Abkühlen, Abziehen der Trägerfolie und Nachtrocknen im Trockenschrank bei 80° C wurde das Blech mit einem deckungsgleichen Vorlagenpaar in Form einer Tasche beidseitig belichtet. Durch Sprühentwicklung unter Verwendung der im Anwendungsbeispiel 5 beschriebenen Entwicklerlösung wurden die belichteten Schichtpartien entwickelt. Die Platte wurde beidseitig mit handelsüblicher Ferrichloridlösung geätzt, bis sie sauber durchgeätzt war. Die Resistschablonen wurden mit einer 4 %igen KOH-Lösung entfernt, und man erhielt ein Formätzteil, das die Vorlage einwandfrei wiedergab.

Anwendungsbeispiel 7

Entsprechend Anwendungsbeispiel 1 wurden 5 lichtempfindliche Druckplatten hergestellt und unbelichtet eine, zwei, drei und vier Stunden in einem Umluftschrank auf 100 °C erwärmt. Nach Ablauf der Zeit wurden die Platten herausgenommen, abgekühlt, 15 Sekunden belichtet und wie im Anwendungsbeispiel 1 beschrieben weiterverarbeitet. Zum Vergleich wurde eine nicht erhitzte Platte belichtet und ebenso verarbeitet.

Während die eine, zwei und drei Stunden erhitzten Platten praktisch keinerlei Unterschied zum Vergleichsmuster zeigten, war der Halbtonstufenkeil bei der 4 Stunden erhitzten Platte um eine Stufe mehr vernetzt.

Dies zeigt die außergewöhnlich gute thermische Stabilität des erfindungsgemäßen Gemischs.

Anwendungsbeispiel 8

Entsprechend Anwendungsbeispiel 1 beschichtete Aluminiumplatten wurden unbelichtet in einem Wärmeschrank (Hotbox) bei einer Temperatur von 56 °C zwei, sechs und dreizehn Wochen aufbewahrt. Nach der jeweiligen Entnahme der Platten wurden diese, wie im Anwendungsbeispiel 1 beschrieben, weiterverarbeitet.

Die in der Hotbox gelagerten Platten zeigten auch nach 3 Monaten weder in der Kopie noch im Druck einen signifikanten Unterschied zu der Originalplatte des Beispiels 1.

Die Lagerstabilität bei erhöhter Temperatur ist demnach sehr gut.

Anwendungsbeispiel 9

Entsprechend Anwendungsbeispiel 1 beschichtete Aluminiumplatten wurden unbelichtet in einem Tropenschrank bei einer Temperatur von 42 °C und einer relativen Feuchtigkeit von 60 % zwei, sechs und dreizehn Wochen aufbewahrt. Nach der jeweiligen Entnahme der Platten wurden diese, wie im Anwendungsbeispiel 1 beschrieben, weiterverarbeitet.

Die im Tropenschrank gelagerten Platten zeigten auch nach 3 Monaten weder in der Kopie noch im Druck einen signifikanten Unterschied zu der Originalplatte des Beispiels 1.

Die Lagerstabilität unter Tropenbedingungen ist demnach sehr gut.

Anwendungsbeispiel 10 und Vergleichsbeispiel 11

Auf eine mechanisch aufgerauhte Aluminiumplatte wurde eine Lösung aus
75 Gt des im Anwendungsbeispiel 5 angegebenen Novolaks,
23,8 Gt eines Polyacetals aus Triethylenglykol und 2-Butyraldehyd,
0,02 Gt Kristallviolettbase und
0,6 Gt der Verbindung Nr. 29 in
24 Gt 2-Methoxy-ethanol und
275 Gt Butanon
aufgeschleudert und getrocknet.

Zum Vergleich wurde die Verbindung 29 in der obigen Rezeptur durch eine äquivalente Menge der Verbindung 2-Trichlormethyl-5-(4-methoxyphenylethenyl)-1,3,4-oxadiazol (Verbindung Nr. 7 der DE-A 28 51 471) ersetzt und damit eine sonst gleiche Platte hergestellt. In beiden Fällen wurde ein Trockenschichtgewicht von 2,2 $g/m^2$ erhalten.

Es wurde durch eine Vorlage belichtet, die einen Stufenkeil und feine Strich- und Rasterelemente aufwies. Die Entwicklung erfolgte mit einer Lösung aus
5,5 Gt Natriummetasilikat x 9 $H_2O$,
3,4 Gt Trinatriumphosphat x 12 $H_2O$ und
0,4 Gt Natriumdihydrogenphosphat in
90,7 Gt entsalztem Wasser.

Bei einer Belichtungszeit von 50 Sekunden und Entwickeln nach 10 Minuten Wartezeit wurden mit der Verbindung Nr. 29 etwa 4 bis 5 vollentwickelte Keilstufen erhalten. Die Wiedergabe der Testelemente erfolgte bis herab in den 10 µm-Bereich. Bei Verwendung der Vergleichsverbindung wurden 2 bis 3 vollentwickelte Keilstufen erhalten; die Auflösung der Testelemente erfolgte bis herab zu etwa 15 bis 20 µm.

**Patentansprüche**

**1.** Verbindungen der allgemeinen Formel I

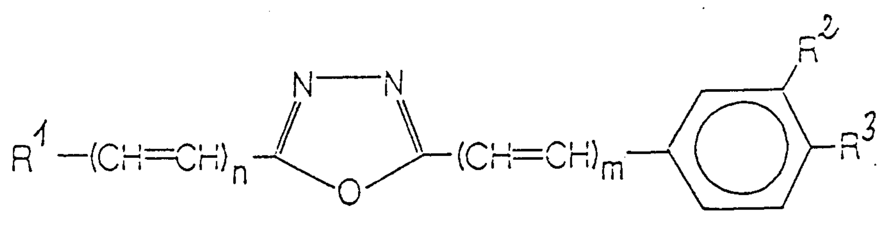

(I)

worin

| | |
|---|---|
| $R^1$ | einen Phenyl-, Naphthyl- oder einen 1- bis 3-kernigen aromatischen N-Heteroylrest, der unsubsituiert oder durch 1 bis 3 Alkyl- oder Alkoxyreste mit 1 bis 10 Kohlenstoffatomen, Perhalogenalkylreste mit 1 bis 6 Kohlenstoffatomen, Nitro-, Cyano-,Carboxyl-, Carbonyl-, Diphenylamino- oder Phenylreste substituiert ist, und |
| $R^2$ und $R^3$ | voneinander verschieden sind und entweder ein Wasserstoffatom oder eine 4,6-Bis-trichlormethyl-s-triazin-2-yl-gruppe und |

n und m voneinander unabhängig die Zahlen 0 oder 1 bedeuten.

2. Verbindungen nach Anspruch 1, dadurch gekennzeichnet, daß $R^1$ ein unsubstituierter oder durch einen der in Anspruch 1 genannten Reste substituierter Phenylrest ist.

3. Verbindungen nach Anspruch 1, dadurch gekennzeichnet, daß $R^2$ ein Wasserstoffatom ist.

4. Lichtempfindliches Gemisch, enthaltend eine lichtempfindliche organische Verbindung (a) mit mindestens einem 4,6-Bis-trichlormethyl-s-triazin-2-ylsubstituenten und eine Verbindung (b), die mit dem Lichtreaktionsprodukt der Verbindung (a) unter Ausbildung eines Produktes zu reagieren vermag, das eine von (b) unterschiedliche Lichtabsorption, Klebrigkeit oder Löslichkeit in einem Entwickler aufweist, dadurch gekennzeichnet, daß die Verbindung (a) eine Verbindung der Formel I gemäß Anspruch 1 ist.

5. Lichtempfindliches Gemisch nach Anspruch 4, dadurch gekennzeichnet, daß die Verbindung (b) eine ethylenisch ungesättigte Verbindung ist, die eine durch freie Radikale ausgelöste Polymerisation einzugehen vermag.

6. Lichtempfindliches Gemisch nach Anspruch 4, dadurch gekennzeichnet, daß die Verbindung (b) mindestens eine durch Säure spaltbare C-O-C-Bindung enthält.

7. Lichtempfindliches Gemisch nach Anspruch 4, dadurch gekennzeichnet, daß die Verbindung (b) durch Säure zur kationischen Polymerisation angeregt zu werden vermag.

8. Lichtempfindliches Gemisch nach Anspruch 4, dadurch gekennzeichnet, daß die Verbindung (b) durch Säure vernetzbar ist.

9. Lichtempfindliches Gemisch nach Anspruch 4, dadurch gekennzeichnet, daß die Verbindung (b) durch Einwirkung von Säure ihren Farbton ändert.

10. Lichtempfindliches Gemisch nach Anspruch 4, dadurch gekennzeichnet, daß es die Verbindung der Formel I in einer Menge von 0,1 bis 15 Gew.-%, bezogen auf seine nichtflüchtigen Bestandteile, enthält.

11. Lichtempfindliches Gemisch nach Anspruch 4, dadurch gekennzeichnet, daß es zusätzlich ein wasserunlösliches polymeres Bindemittel enthält.

12. Lichtempfindliches Gemisch nach Anspruch 11, dadurch gekennzeichnet, daß das Bindemittel in wäßrig-alkalischen Lösungen löslich ist.

13. Verfahren zur Herstellung einer Verbindung der Formel I gemäß Anspruch 1, dadurch gekennzeichnet, daß man eine Verbindung der Formel II

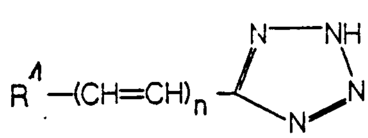

(II)

mit einem Carbonsäurehalogenid der Formel III

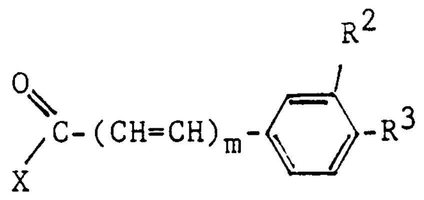

(III)

umsetzt, wobei X ein Halogenatom ist und die Symbole $R^1$, $R^2$, $R^3$, m und n die im Anspruch 1 angegebene Bedeutung haben.

**14.** Verfahren nach Anspruch 13, dadurch gekennzeichnet, daß man die Umsetzung in einer heterocyclischen Base als Reaktionsmedium bei einer Temperatur zwischen 50 und 150 °C durchführt.

**Claims**

**1.** A compound of the formula I

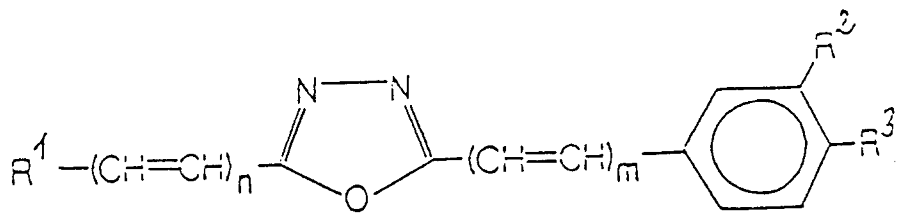

(I)

wherein

| | |
|---|---|
| $R^1$ | denotes a phenyl or napthyl radical or a mono- to trinuclear aromatic N-heteroyl radical, which is unsubstituted or substituted by 1 to 3 alkyl or alkoxy radicals having 1 to 10 carbon atoms, perhaloalkyl radicals having 1 to 6 carbon atoms, nitro, cyano, carboxyl, carbonyl, diphenylamino or phenyl radicals, and |
| $R^2$ and $R^3$ | are different from each other and either denote a hydrogen atom or a 4,6-bistrichloromethyl-s-triazin-2-yl group, and |
| n and m, | independently of each other, denote the number 0 and 1. |

**2.** A compound as claimed in claim 1, wherein $R^1$ is a phenyl radical which is unsubstituted or substituted by one of the radicals mentioned in claim 1.

**3.** A compound as claimed in claim 1, wherein $R^2$ is a hydrogen atom.

**4.** A photosensitive composition, comprising a photosensitive organic compound (a) having at least one 4,6-bis-trichloromethyl-s-triazin-2-yl substituent and a compound (b) which is capable of reacting with the photoreaction product of compound (a) to form a product of which the light absorption, tackiness or solubility in a developer differs from that of (b), wherein compound (a) is a compound of the formula I as claimed in claim 1.

**5.** The photosensitive composition as claimed in claim 4, wherein compound (b) is an ethylenically unsaturated compound which is capable of undergoing a polymerization initiated by free radicals.

**6.** The photosensitive composition as claimed in claim 4, wherein compound (b) contains at least one C-O-C bond which can be split by acid.

**7.** The photosensitive composition as claimed in claim 4, wherein compound (b) can be induced to cationic polymerization by acid.

8. The photosensitive composition as claimed in claim 4, wherein compound (b) can be crosslinked by acid.

9. The photosensitive composition as claimed in claim 4, wherein compound (b) changes its color shade under the action of acid.

10. The photosensitive composition as claimed in claim 4, which comprises the compound of the formula I in a quantity from 0.1 to 15% by weight, based on its nonvolatile constituents.

11. The photosensitive composition as claimed in claim 4, which additionally comprises a water-insoluble polymeric binder.

12. The photosensitive composition as claimed in claim 11, wherein the binder is soluble in aqueous-alkaline solutions.

13. A process for preparing a compound of the formula I as claimed in claim 1, wherein a compound of the formula II

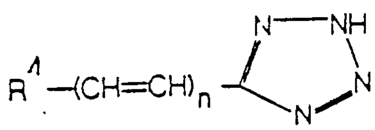

(II)

is reacted with a carboxylic acid halide of the formula III

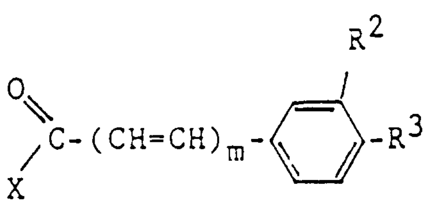

(II)

X being a halogen atom and the symbols $R^1$, $R^2$, $R^3$, m and n being as defined in claim 1.

14. The process as claimed in claim 13, wherein the reaction is carried out in a heterocyclic base as the reaction medium, at a temperature between 50 and 150 °C.

## Revendications

1. Composés de formule générale I

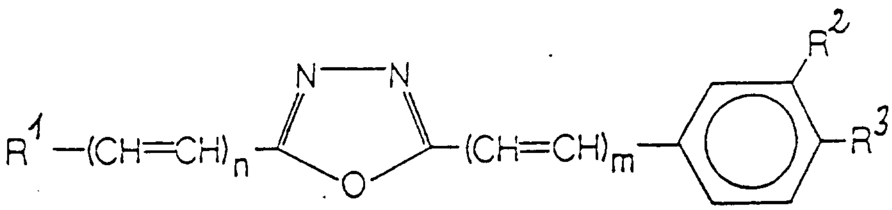

(I)

dans laquelle

$R^1$ représente un radical phényle, naphtyle ou N-hétérocyclique de 1 à 3 noyaux aromatiques, non substitué ou substitué par 1 à 3 radicaux alkyle ou alcoxy ayant 1 à 10 atomes de carbone, alkyle perhalogéné ayant 1 à 6 atomes de carbone, nitro, cyano, carboxyle, carbonyle, diphénylamino ou phényle, et

$R^2$ et $R^3$, différents l'un de l'autre, représentent soit un atome d'hydrogène soit un groupe 4,6-bis-trichlorométhyl-S-triazine-2-yle ; et

n et m indépendamment l'un de l'autre, représentent l'entier 0 ou 1.

**2.** Composés tels que revendiqués dans la revendication 1, dans lesquels $R^1$ est un radical phényle non substitué ou substitué par l'un des radicaux nommés dans la revendication 1.

**3.** Composés tels que revendiqués dans la revendication 1, dans lesquels $R^2$ est un atome d'hydrogène.

**4.** Composition photosensible, comprenant un (a) composé organique photosensible avec au moins un substituant 4,6-bis-trichlorométhyl-S-triazine-2-yle et un (b) composé qui est capable de réagir avec le produit de la photoréaction provenant du composé (a), formant un produit dont l'absorption de la lumière, le degré d'adhérence ou la solubilité dans un révélateur diffèrent de celui de (b), caractérisé en ce que le composé (a) est un composé de formule I, tel que revendiqué dans la revendication 1.

**5.** Composition photosensible telle que revendiquée dans la revendication 4, dans laquelle le composé (b) est un composé éthylènique insaturé capable de subir une polymérisation initiée par des radicaux libres.

**6.** Composition photosensible telle que revendiquée dans la revendication 4, dans laquelle le composé (b) contient au moins une liaison C-O-C pouvant être élevée par un acide.

**7.** Composition photosensible telle que revendiquée dans la revendication 4, dans laquelle la polymérisation cationique du composé (b) peut être induite par un acide.

**8.** Composition photosensible telle que revendiquée dans la revendication 4, dans laquelle le composé (b) peut être réticulé par un acide.

**9.** Composition photosensible telle que revendiquée dans la revendication 4, dans laquelle le composé (b) change de nuance de couleur sous l'action d'un acide.

**10.** Composition photosensible telle que revendiquée dans la revendication 4, caractérisée en ce qu'elle contient le composé de la formule I en une quantité allant de 0,1 à 15 % en poids, par rapport à ses constituants non volatils.

**11.** Composition photosensible telle que revendiquée dans la revendication 4, caractérisée en ce qu'elle contient en plus un liant polymérique insoluble dans l'eau.

**12.** Composition photosensible telle que revendiquée dans la revendication 11, caractérisée en ce que le liant est soluble dans des solutions alcalines aqueuses.

**13.** Procédé de préparation d'un composé de formule I tel que revendiqué dans la revendication 1, dans lequel on fait réagir un composé de formule II

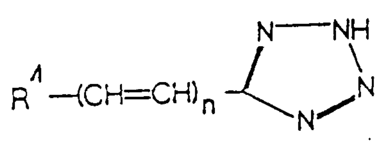 (II)

avec un halogénure d'un acide carboxylique de formule III

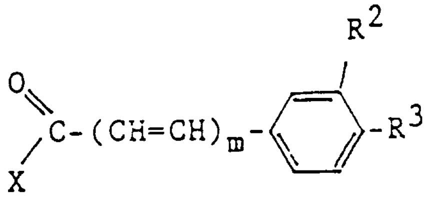 (III)

où X est un atome d'halogène et les symboles $R^1$, $R^2$, $R^3$, m et n sont tels que définis dans la revendication 1.

**14.** Procédé tel que revendiqué dans la revendication 13, dans lequel la réaction est effectuée dans une base hétérocyclique en tant que milieu de réaction, à une température entre 50 et 150 °C.